# EUROPEAN PATENT SPECIFICATION

(11) **EP 3 786 176 B1**
(45) Date of publication and mention of the grant of the patent: **01.07.2026**
(21) Application number: 20187535.8
(22) Date of filing: 27.10.2016
(51) Int. Cl.: C07K 14/47, C12N 9/64

(54) **GENE THERAPY**
GENTHERAPIE
THÉRAPIE GÉNIQUE

(30) Priority: 28.10.2015 GB 201519086
(43) Date of publication of application: 03.03.2021
(62) Divisional of application: 17210269.1
(73) Proprietor: Syncona IP Holdco Limited, London WC1B 3SR (GB)
(72) Inventor: GROENDAHL, Christian, 2800 Kgs. Lyngby (DK); FUNNELL, Tim, London NW1 2BE (GB); HOLLOWOOD, Chris, London NW1 2BE (GB)
(74) Representative: D Young & Co LLP

(56) References cited:
- WO-A1-2014/172560
- WO-A1-2014/207190
- WO-A1-2017/194912
- US-A1- 2010 120 665
- US-A1- 2014 234 275
- M T BIRKE ET AL: "AAV-mediated expression of human PRELP inhibits complement activation, choroidal neovascularization and deposition of membrane attack complex in mice", GENE THERAPY, vol. 21, no. 5, 27 March 2014 (2014-03-27), GB, pages 507 - 513, XP055336523, ISSN: 0969-7128, DOI: 10.1038/gt.2014.24
- ANDERSON D H ET AL: "The pivotal role of the complement system in aging and age-related macular degeneration: Hypothesis re-visited", PROGRESS IN RETINAL AND EYE RESEARCH, OXFORD, GB, vol. 29, no. 2, 1 March 2010 (2010-03-01), pages 95 - 112, XP026929884, ISSN: 1350-9462, [retrieved on 20091202], DOI: 10.1016/J.PRETEYERES.2009.11.003

## Description

### FIELD OF THE INVENTION

The present invention relates to compounds for use in the gene therapy of eye diseases. More specifically, the invention relates to adeno-associated viral (AAV) vectors, for use in the treatment or prevention of age-related macular degeneration (AMD), wherein the vectors enable delivery of Factor I or fragments thereof to the eye.

### BACKGROUND TO THE INVENTION

The macula is a small area in the retina of the eye, approximately 3 to 5 millimetres in size, adjacent to the optic nerve. It is the most sensitive area of the retina and contains the fovea, a depressed region that allows for high visual acuity and contains a dense concentration of cones, the photoreceptors that are responsible for colour vision.

Age-related macular degeneration (AMD) is the most common cause of functional blindness in developed countries for those over 50 years of age (Seddon, J M. Epidemiology of age-related macular degeneration. In: Ogden, T E, et al., eds. Ryan S J, ed-in-chief. Retina Vol II. 3rd ed. St. Louis, Mo.: Mosby; 2001:1039-50). AMD is associated with neovascularisation originating from the choroidal vasculature and extending into the subretinal space. In addition, AMD is characterized by progressive degeneration of the retina, retinal pigment epithelium (RPE), and underlying choroid (the highly vascular tissue that lies beneath the RPE, between the retina and the sclera).

A variety of factors including oxidative stress, inflammation with a possible autoimmune component, genetic background (e.g., mutations), and environmental or behavioural factors such as smoking and diet may contribute to the pathogenesis of AMD.

The clinical progression of AMD is characterised in stages according to changes in the macula. The hallmark of early AMD is drusen, which are accumulations of extracellular debris underneath the retina and appear as yellow spots in the retina on clinical exam and on fundus photographs. Drusens are categorised by size as small (<63 µm), medium (63-124 µm) and large (>124 µm). They are also considered as hard or soft depending on the appearance of their margins on ophthalmological examination. While hard drusens have clearly defined margins, soft ones have less defined and fluid margins. The Age-related Eye Disease Study (AREDS) fundus photographic severity scale is one of the main classification systems used for this condition.

AMD has been classified into "dry" and "wet" (exudative, or neovascular) forms. Dry AMD is more common than wet AMD, but the dry form can progress to the wet form, and the two occur simultaneously in a significant number of cases. Dry AMD is typically characterized by progressive apoptosis of cells in the RPE layer, overlying photoreceptor cells, and frequently also the underlying cells in the choroidal capillary layer. Confluent areas of RPE cell death accompanied by overlying photoreceptor atrophy are referred to as geographic atrophy. Patients with this form of AMD experience a slow and progressive deterioration in central vision.

Wet AMD is characterized by bleeding and/or leakage of fluid from abnormal vessels that have grown from the choroidal vessels (choriocapillaris) beneath the RPE and the macula, which can be responsible for sudden and disabling loss of vision. It has been estimated that much of the vision loss that patients experience is due to such choroidal neovascularization (CNV) and its secondary complications. A subtype of neovascular AMD is termed retinal angiomatous proliferation (RAP). Here, angiomatous proliferation originates from the retina and extends posteriorly into the subretinal space, eventually communicating in some cases with choroidal new vessels.

The complement system (CS) has been implicated in early AMD pathogenesis based on the identification of CS components in drusen from eyes of AMD patients. In AMD, at least 129 types of drusen-deposited proteins have been identified, including different apolipoprotein types (E, B, or A-I), several amyloid peptides (P, Aβ, or SA-1), TIMP-3, serum albumin, and certain proteins associated with cellular function (e.g. ATP synthase β subunit, scavenger receptor B2, and retinol dehydrogenase). AMD-derived drusen also contain almost all of the complement proteins, including regulatory proteins (CFH, complement receptor 1 (CR1), vitronectin, and clusterin), the products of CS activation and degradation (C1q, C3, C3a, C3b, and C5a), and members of the terminal CS pathway comprising the MAC components (i.e. 5, 6, 8 (α, β, and γ), and 9) in the separated and complex form. Accumulating drusen may activate the CS, trigger the local production of inflammatory mediators, and attract leukocytes that in turn augment the local inflammatory state present in AMD.

Current treatment options for AMD include photodynamic therapy with benzoporphyrin (Arch Ophthalmol. 1999;117:1329-1345) and a number of therapies which target the Vascular Endothelial Growth Factor (VEGF) pathway. Examples of such VEGF-targeted therapies include the aptamer pegaptanib (N Engl J Med. 2004;351:2805-2816) and antibodies such as ranibizumab (N Engl J Med. 2006 Oct 5; 355(14):1432-44) and bevacizumab (BMJ. 2010 Jun 9; 340:c2459.). However, not all patients respond to treatment with an anti-VEGF antibody and either do not recover vision or progress to registered blindness.

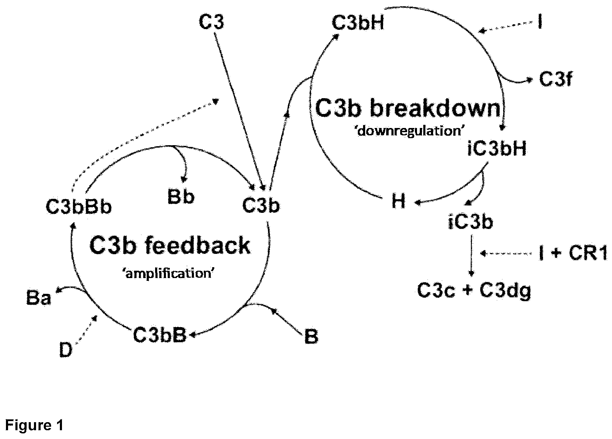

A therapy for the treatment of geographic atrophy has been developed and is currently in a phase III clinical study (MAHALO study by Genentech/Roche). Lampalizumab is a humanised monoclonal inhibitory antibody to complement Factor D, administered by intravitreal injection to stop the rate of progression of geographic atrophy. As show in Figure 1, Factor D is part of the C3b feedback ('amplification') cycle. Factor D is present in very low serum concentrations and is an essential factor for the alternative pathway. Nevertheless, due to its small size (27 kDa), Factor D is rapidly cleared out by the kidneys and quickly re-synthesised. The therapy requires monthly intravitreal injections.

WO 2017/194912 A1 discloses a method for preventing, treating, or ameliorating a complement-mediated disorder in a subject in need thereof, which comprises administering to the subject a recombinant viral vector comprising nucleic acid encoding Factor I, or a fragment or derivative thereof that retains C3b-inactivating and iC3b-degradation activity, such that a therapeutically effective amount of the encoded Factor I, or the fragment or derivative thereof, is expressed from the nucleic acid in the subject, thereby increasing the level of C3b-inactivating and iC3b- degradation activity in the subject. US 2014/234275 A1 discloses methods and systems for the treatment for ALS incorporating stem cells harvested from the subject to be treated. WO 2014/172560 A1 discloses a method of treating macular degeneration in a mammalian subject comprising administering to the diseased eye of the subject a composition comprising a recombinant vector encoding an IL17 inhibitor. Birke et al. (2014) Gene Therapy 21, 507-513 discloses that AAV-mediated expression of human PRELP inhibits complement activation, choroidal neovascularization and deposition of membrane attack complex in mice.

There is a need in the art for new approaches to treat AMD.

### SUMMARY OF THE INVENTION

The present inventors now provide an approach for modulating the complement system which is useful, for example, in the treatment of AMD. The inventors provide Factor I delivered by gene therapy with the aim of negatively regulating the complement C3b feedback cycle through targeting of the breakdown cycle (Figure 1). The resulting rebalancing of the feedback loop of the alternative pathway will promote C3b and iC3b breakdown and thus remove major disease factors in complement-mediated disorders, particularly disorders that have an underlying defect in alternative pathway regulation.

In one aspect, the invention provides an adeno-associated viral (AAV) vector comprising a nucleotide sequence encoding Factor I or a fragment thereof capable of cleaving C3b into iC3b, wherein the nucleotide sequence encoding Factor I or the fragment thereof is operably linked to a polyadenylation site. Also described herein, but not claimed, are an adeno-associated viral (AAV) vector comprising a nucleotide sequence encoding Factor H or a fragment or derivative thereof, an adeno-associated viral (AAV) vector comprising a nucleotide sequence encoding an anti-Factor D antibody, and an adeno-associated viral (AAV) vector comprising a nucleotide sequence encoding an anti-complement component 5 (C5) antibody.

In one embodiment, the nucleotide sequence encoding Factor I or fragment thereof comprises a sequence selected from the group consisting of:
(a) a nucleotide sequence encoding an amino acid sequence that has at least 70% identity to SEQ ID NO: 1 or 9;
(b) a nucleotide sequence that has at least 70% identity to SEQ ID NO: 2 or 8; and
(c) the nucleotide sequence of SEQ ID NO: 2 or 8.

Preferably, the nucleotide sequence encoding Factor I or fragment thereof encodes a protein with the natural activity of Factor I (e.g. the protein represented by SEQ ID NO: 1 or 9). For example, the nucleotide sequence encoding Factor I or fragment or derivative thereof may encode a protein with the ability to process C3b and iC3b into inactive degradation products. Put another way, the Factor I or fragment thereof preferably retains C3b-inactivating and iC3b-degradation activity.

In a preferred embodiment, the nucleotide sequence encoding Factor I or fragment thereof encodes a protein with C3b-inactivating and iC3b-degradation activity.

In another aspect, the invention provides an isolated cell transfected with and comprising the AAV vector of the invention.

In another aspect, the invention provides a pharmaceutical composition comprising the AAV vector of the invention or the isolated cell of the invention in combination with a pharmaceutically acceptable carrier, diluent or excipient. In a preferred embodiment, the pharmaceutical composition is for intraocular administration.

In one embodiment, the AAV vector comprises a chicken beta-actin (CBA) promoter, for example operably linked to the nucleotide sequence encoding Factor I or fragment thereof. In one embodiment, the AAV vector comprises a CAG promoter, for example operably linked to the nucleotide sequence encoding Factor I or fragment thereof. In one embodiment, the AAV vector comprises a promoter with the nucleotide sequence of SEQ ID NO: 5, for example operably linked to the nucleotide sequence encoding Factor I or fragment thereof.

In one embodiment, the AAV vector comprises a cytomegalovirus (CMV) enhancer element, for example operably linked to the nucleotide sequence encoding Factor I or fragment thereof.

In one embodiment, the AAV vector comprises a Bovine Growth Hormone poly-A signal, for example operably linked to the nucleotide sequence encoding Factor I or fragment thereof, preferably a Bovine Growth Hormone poly-A signal having the nucleotide sequence of SEQ ID NO: 6.

In one embodiment, the AAV vector comprises a woodchuck hepatitis post-transcriptional regulatory element (WPRE), for example operably linked to the nucleotide sequence encoding Factor I or fragment thereof, preferably a WPRE having the nucleotide sequence of SEQ ID NO: 7.

In a preferred embodiment, the AAV vector of the invention is in the form of a viral particle.

In a preferred embodiment, the AAV viral particle comprises an AAV2 genome and AAV2 capsid proteins. Preferably, the nucleotide sequence encoding Factor I or fragment thereof is operably linked to a CAG promoter, preferably a promoter with the nucleotide sequence of SEQ ID NO: 5.

The AAV vector, isolated cell or pharmaceutical composition of the invention may be used to treat or prevent an ocular disorder.

In one embodiment, the invention provides the AAV vector, isolated cell or pharmaceutical composition of the invention for use in treating or preventing a complement-mediated disorder of the eye.

In one embodiment, the disorder is associated with over-activity of the complement C3b feedback cycle and/or under-activity of the C3b breakdown cycle (see Figure 1). In one embodiment, the disorder is age-related macular degeneration (AMD) or diabetic retinopathy. In a preferred embodiment, the disorder is AMD, preferably dry AMD.

In one embodiment, the use is for treating or preventing a disorder in a subject:
(a) having lower than normal Factor I activity or concentration in the eye and/or serum, preferably having a concentration of, or activity equivalent to, 0-30 or 0-20 or 0-10 µg/mL in serum; and/or
(b) being heterozygous or homozygous for an age-related macular degeneration (AMD)-associated SNP, preferably a rare Factor I variant.

In one embodiment, the use is for treating or preventing a disorder in a subject:
(a) having a normal level of Factor I activity or concentration in the eye and/or serum, preferably at least 30 µg/mL, such as 30-40 µg/mL in serum; and/or
(b) not carrying a rare Factor I variant allele.

In another aspect, the invention provides the AAV vector, isolated cell or pharmaceutical composition of the invention for use in treating or preventing age-related macular degeneration (AMD). The AMD may, for example, be dry AMD. In a preferred embodiment, the AMD is dry AMD.

In another aspect, the invention provides the AAV vector, isolated cell or pharmaceutical composition of the invention for use in treating or preventing diabetic retinopathy.

In one embodiment, the formation of geographic atrophy is prevented or reduced. In another embodiment, the amount of geographic atrophy is reduced.

In one embodiment, the progression of geographic atrophy is slowed. Preferably, there is at least a 10%, 20%, 30%, 40%, 50%, 60%, 70%, 80% or 90% reduction in the increase in geographic atrophy area over the 12 months following administration to a treated eye of a subject, relative to an untreated eye over the same period.

In another aspect, the invention provides the AAV vector, isolated cell or pharmaceutical composition of the invention for use in improving or restoring vision or visual acuity, for example in a subject suffering from an eye disorder, such as an eye disorder disclosed herein. In another aspect, the invention provides the AAV vector, isolated cell or pharmaceutical composition of the invention for use in mitigating loss of vision or visual acuity, for example a loss of vision or visual acuity associated with an eye disorder, such as an eye disorder disclosed herein.

In another aspect, the invention provides the AAV vector, isolated cell or pharmaceutical composition of the invention for use in improving or restoring reading speed in a subject, for example in a subject suffering from an eye disorder, such as an eye disorder disclosed herein. In another aspect, the invention provides the AAV vector, isolated cell or pharmaceutical composition of the invention for use in mitigating reduction in reading speed in a subject, for example a reduction in reading speed associated with an eye disorder, such as an eye disorder disclosed herein.

In another aspect, the invention provides the AAV vector, isolated cell or pharmaceutical composition of the invention for use in reducing or preventing loss of photoreceptors and/or the retinal pigment epithelium (RPE), for example a loss of photoreceptors and/or the RPE associated with an eye disorder, such as an eye disorder disclosed herein.

In a preferred embodiment, the AAV vector, isolated cell or pharmaceutical composition for use according to the invention is administered intraocularly. The inventors recognise that such local administration of the therapy provides a means practically to achieve the required levels of the complement factor for treating or preventing the complement-mediated disorder of the eye, for example AMD.

In one embodiment, the AAV vector, isolated cell or pharmaceutical composition of the invention is administered to the eye of a subject by subretinal, direct retinal, suprachoroidal or intravitreal injection.

In a particularly preferred embodiment, the AAV vector, isolated cell or pharmaceutical composition of the invention is administered to the eye of a subject by subretinal injection.

In one embodiment, administration of the AAV vector, isolated cell or pharmaceutical composition of the invention thereby increases the level of C3b-inactivating and iC3b-degradation activity in the subject, in particular in the eye, such as in the RPE, of the subject. In another embodiment, administration of the AAV vector, isolated cell or pharmaceutical composition of the invention thereby increases the level of C3b-inactivating and iC3b-degradation activity in the subject, in particular in the eye, such as in the RPE, of the subject to a level that exceeds a normal level in the eye.

Also described herein, but not claimed, is a method of treating or preventing a complement-mediated disorder of the eye comprising administering the AAV vector, isolated cell or pharmaceutical composition of the invention to a subject in need thereof.

In one embodiment, the disorder is associated with over-activity of the complement C3b feedback cycle and/or under-activity of the C3b breakdown cycle. In one embodiment, the disorder is age-related macular degeneration (AMD) or diabetic retinopathy. In a preferred embodiment, the disorder is AMD, preferably dry AMD.

Also described herein, but not claimed, is a method of treating or preventing age-related macular degeneration (AMD) comprising administering the AAV vector, isolated cell or pharmaceutical composition of the invention to a subject in need thereof. The AMD may, for example, be dry AMD. In a preferred embodiment, the AMD is dry AMD.

Also described herein, but not claimed, is a method of treating or preventing diabetic retinopathy comprising administering the AAV vector, isolated cell or pharmaceutical composition of the invention to a subject in need thereof.

The subject may, for example, have been diagnosed with AMD or be at risk from acquiring AMD.

In a preferred embodiment, the AAV vector, isolated cell or pharmaceutical composition is administered intraocularly.

In one embodiment, the AAV vector, isolated cell or pharmaceutical composition is administered to the eye of a subject by subretinal, direct retinal, suprachoroidal or intravitreal injection.

In a particularly preferred embodiment, the AAV vector, isolated cell or pharmaceutical composition is administered to the eye of a subject by subretinal injection.

Also described herein, but not claimed, is the use of the AAV vector, isolated cell or pharmaceutical composition of the invention for manufacturing a medicament for treating or preventing a complement-mediated disorder of the eye.

In one embodiment, the disorder is associated with over-activity of the complement C3b feedback cycle and/or under-activity of the C3b breakdown cycle. In one embodiment, the disorder is age-related macular degeneration (AMD) or diabetic retinopathy. In a preferred embodiment, the disorder is AMD, preferably dry AMD.

Also described herein, but not claimed, is the use of the AAV vector, isolated cell or pharmaceutical composition of the invention for manufacturing a medicament for treating or preventing age-related macular degeneration (AMD). In a preferred embodiment, the AMD is dry AMD.

Also described herein, but not claimed, is the use of the AAV vector, isolated cell or pharmaceutical composition of the invention for manufacturing a medicament for treating or preventing diabetic retinopathy.

In one embodiment, the AAV vector of the invention does not comprise a hAAT promoter.

In one embodiment, the AAV vector of the invention does not comprise an ApoR enhancer.

In another embodiment, the AAV vector of the invention does not comprise two ApoR enhancers.

In one embodiment, the AAV vector of the invention does not comprise an AAV2 genome and an AAV8 capsid protein, i.e. the AAV vector of the invention is not an AAV2/8 vector.

In one embodiment, the AAV vector, isolated cell or pharmaceutical composition of the invention is not administered systemically. In another embodiment, the AAV vector, isolated cell or pharmaceutical composition of the invention is not administered intravenously.

### DESCRIPTION OF THE DRAWINGS

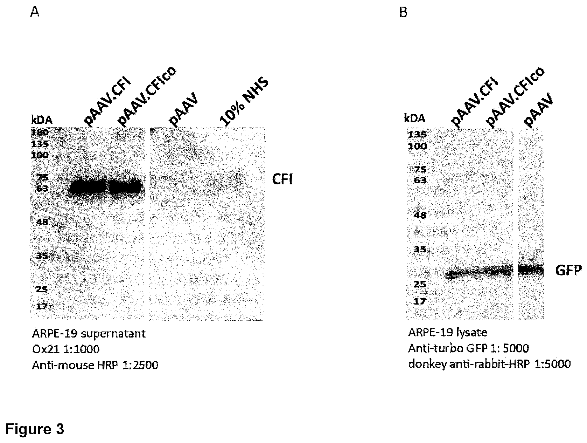

**Figure 1**
   C3b feedback (amplification) and breakdown (down-regulation) cycles of the alternative pathway of vertebrate complement ("I" = Factor I; "H" = Factor H; "B" = Factor B; and "D" = Factor D).
**Figure 2**
   An agarose gel of restriction digests of CFI and CFlco. The 1752 bp band of CFI was excised and cloned into the pAAV-CBA- WPRE-bGHpA backbone.
**Figure 3**
   Immunoblotting of CFI (Figure 3A) and of GFP (Figure 3B). 3A: CFI appears as a 70kDa band (non-reduced) and was expressed at equal rates after transfection of ARPE-19 with pAAV.CFI or pAAV.CFlco. No CFI was expressed after transfection with pAAV. 10% normal human serum (NHS) was used as a positive control for CFI immunoblotting. 3B: Transfection efficiency was analysed by co-transfection of ARPE-19 cells with pCMV.GFP. GFP appears as a 30kDa band and the immunoblot confirmed that cells have been transfected at similar efficiencies.
**Figure 4**
   Immunoblotting of CFI in supernatant of virus transduced HEK-293 and ARPE-19 cell lines. 4A: Supernatant was loaded under non-reducing conditions and CFI was detected with a mouse monoclonal antibody to human CFI (OX21, Thermo Fisher Scientific) and a donkey anti-mouse IgG HRP conjugated antibody (Abcam). CFI and CFlco were expressed in both cell lines. Transduction of cell lines with AAV.GFP served as a negative control while 0.5µg of plasma purified human CFI (called "CFlpl" herein) (Comptech) served as a positive control. 4B: Supernatant was loaded under reducing conditions and CFI was detected with a goat antiserum to human CFI (Comptech) and rabbit anti-goat IgG (whole molecule) - Peroxidase antibody (Sigma). CFI appeared at 80kDa (pro-enzyme), 50kDa (processed; heavy chain) and 35kDa (processed; light chain). AAV.GFP served as a negative control while 0.5µg of plasma purified human CFI (Comptech) and 10% normal human serum (called "NHS" herein) served as a positive control. CFI and CFlco were expressed in both cell lines.
**Figure 5**
   A representative result of a C3b cleavage assay. Lane 1 shows C3b incubated with CFH only. Lane 2 shows C3b incubated with CFH and CFlpl. C3b is degraded by CFlpl to iC3b and C3dg. Lane 3 shows C3b incubated with CFH and supernatant from HEK-293 transduced with AAV.CFI. Lane 4-5 show C3b incubated with CFH and immunoprecipitated CFI (designated as "IP CFI") from ARPE-19 cells transduced with either AAV.CFI (lane 4) or AAV.CFlco (lane 5). Lane 6-7 show C3b incubated with CFH and immunoprecipitated CFI from HEK-293 transduced with either AAV.CFI (lane 6) or AAV.CFlco (lane 7).
**Figure 6**
   Immunoblotting of CFI secreted from transwell cultured ARPE-19 cells. Cells were not differentiated to hexagonal cells however they were cultured as a confluent monolayer of cells and cell division was reduced to a minimum by addition of 1% serum medium. 6A: Supernatant from both compartments was loaded under non reducing conditions and western blot analysis was performed using a mouse monoclonal to CFI (Ap=apical compartment and Bl=basolateral compartment). It is shown that CFI is being expressed from a confluent monolayer of cells and that secreted protein is detected in both compartments. 6B: Hoechst staining of nuclei was performed to confirm presence of a monolayer of cells (Staining was performed after harvesting the supernatant).
**Figure 7**
   CFI protein expression of pooled samples analysed by immunoblotting. CFI is expressed at detectable levels at all doses and from both AAV.CFI and AAV.CFlco. β-actin was loaded as a loading control. 7A: 40µg protein lysate were loaded under reducing conditions and CFI was detected with a polyclonal goat antiserum to human CFI. CFI is detected as 80kDa (pro-enzyme), 50kDa (processed; heavy chain) and 35kDa (processed; light chain). These bands correspond to the expected size of CFI and confirm processing, i.e. presence of heavy and light chain. 7B: The same amount of protein lysate was also loaded for lysate samples of eyes injected with 10⁹gc/eye of AAV.CFlco or uninjected eyes. CFI was detected with a mouse monoclonal to CFI (left, non-reducing gel) and goat antiserum to CFI (right, reducing gel). The non reducing gel (left) detects CFI as a band at 75kDa in injected animals and no band is detected in the uninjected eye. In the reducing gel (right) CFI appears as 80kDa (pro-enzyme), 50kDa (processed; heavy chain) and 35kDa (processed; light chain).
**Figure 8**
   Gene expression analysis by qPCR.
**Figure 9**
   hCFI localisation in sham (A-C), AAV.CFI (D-F) and AAV.CFlco (G-H) injected eyes. Retinal sections were double labelled with fibronectin (A,D and G) and hCFI (B,E and H). Nuclei were stained with DAPI and are shown in merge (C, F and I). Scl: Sclera, RPE: Retinal Pigment Epithelium, OS: Outer Segment of photoreceptors, IS: Inner Segment of photoreceptors, OPL: Outer Plexiform Layer, GCL: Ganglion Cell Layer, NFL: Nerve Fiber Layer, Magnification: 20X, Scale bar: 50 µm.
**Figure 10**
   hCFI localisation in sham (A-C) and AAV.CFI (D-F) injected eyes: higher magnification of RPE. Retinal sections were double labelled with fibronectin (A and D) and hCFI (B and E). Nuclei were stained with DAPI and are shown in merge (C and F). Scl: Sclera, Bru: Bruch's membrane, Cho: Choriocapillaris, RPE: Retinal Pigment Epithelium, IPM: Inter Photoreceptor Matrix. Magnification: 189X, Scale bar: 10 µm. Vesicular staining is depicted with arrows, RPE microvilli are depicted at the inferior edge of RPE with stars.
**Figure 11**
   hCFI localisation in sham (A-C) and AAV.CFI (D-F) injected eyes: higher magnification of photoreceptor layers. Retinal sections were double labelled with fibronectin (A and D) and hCFI (B and E). Nuclei were stained with DAPI and are shown in merge (C and F). IPM: Inter Photoreceptor Matrix, OS: Outer Segment, IS: Inner Segment, ONL: Outer Nuclear Layer. Magnification: 189X, Scale bar: 10 µm.
**Figure 12**
   hCFI localisation in sham (A-C) and AAV.CFI (D-F) injected eyes: higher magnification of the Outer Plexiform Layer (OPL). Retinal sections were double labelled with fibronectin (A and D) and hCFI (B and E). Nuclei were stained with DAPI and are shown in merge (C and F). ONL: Outer Nuclear Layer, INL: Inner Nuclear Layer. Magnification: 189X, Scale bar: 10 µm. Horizontal cells staining is depicted with arrows.
**Figure 13**
   hCFI localisation in sham (A-C) and AAV.CFI (D-F) injected eyes: higher magnification of the Ganglion Cell Layer (GCL). Retinal sections were double labelled with fibronectin (A and D) and hCFI (B and E). Nuclei were stained with DAPI and are shown in merge (C and F). IPL: Inner Plexiform Layer, NFL: Nerve Fiber Layer. Magnification: 189X, Scale bar: 10 µm.
**Figure 14**
   hCFI localisation in sham (A-C), AAV.CFI (D-F) and AAV.CFlco (G-H) injected eyes. Whole mount Retinal Pigment Epithelium were double labelled with fibronectin (A,D and G) and hCFI (B,E and H). Magnification: 40X, Scale bar: 30 µm.

### DETAILED DESCRIPTION OF THE INVENTION

### Complement System

The complement system is an integral part of the humoral immune system and is involved in tissue inflammation, cell opsonization, and cytolysis. In provides protection against microorganisms and mediates the clearance of exogenous and endogenous cellular debris from the host tissues.

The complement system cascade is comprised of four activation pathways. All of the pathways ultimately end in the central cleavage of C3 factor and in the generation of its active fragments C3a and C3b. C3a is the anaphylatoxin that triggers a range of chemotactic and proinflammatory responses, such as recruitment of inflammatory cells and increased microvasculature permeability, whereas C3b is responsible for opsonization of foreign surfaces covalently attached to C3b. Opsonization with activated C3 fragments (C3b and iC3b) fulfils three major functions: (i) cell debris elimination by phagocytic cells (e.g., macrophages or microglia) and the stimulation of the adaptive immune system (B and T cells); (ii) amplification of complement activation via the formation of a surface-bound C3 convertase; and (iii) assemblage of the C5 convertase.

Assemblage of the C5 convertase is responsible for C5 cleavage, which results in the formation of the cytolytic membrane attack complex (MAC) capable of generating perforations in the cell membrane, thereby promoting cell lysis and the elimination of unnecessary cells. Through all of these activities, the innate complement cascade supports and promotes the function of downstream mechanisms of the immune system that protect the integrity of the host tissue. Overall, complement system pathway activation results in a proinflammatory response, including MAC generation, which mediates cell lysis, the release of chemokines to attract inflammatory cells to the site of damage, and the enhancement of capillary permeability to promote extravasation of infiltrating leukocytes. Under physiological conditions, complement activation is effectively controlled by the coordinated action of soluble and membrane-associated complement regulatory molecules (CRMs). Soluble complement regulators, such as C1-inhibitor, anaphylatoxins inhibitor, C4b binding protein (C4BP), complement factor H (CFH), complement factor I (CFI), clusterin, and vitronectin, restrict the action of complement in human tissues at multiple sites of the cascade reaction. In addition, each individual cell is protected against the attack of homologous complement by surface proteins, such as the complement receptor 1 (CR1, CD35), the membrane cofactor protein (CD46), and glycosylphosphatidylinositol-anchored proteins, such as decay-accelerating factor (CD55) or CD59 molecule. Of note, host cells and tissues that are inadequately protected from complement attack might be subjected to bystander cell lysis.

The present disclosure relates to the treatment or prevention of a complement-mediated disorder of the eye. For example, the complement-mediated disorder may be a disorder associated with a defect in alternative pathway regulation, and in particular with over-activity of the complement C3b feedback cycle and/or under-activity of the C3b breakdown cycle.

In one embodiment, prior to administration of the AAV vector, isolated cell or pharmaceutical composition of the invention, the subject has low levels (e.g. lower than normal levels) of Factor I activity, for example low levels of Factor I activity in the eye and/or low serum levels of Factor I activity. The sub-normal level of Factor I activity may be due to sub-normal expression of normally-functioning Factor I, or at least partial (e.g. heterozygous) expression (at normal or sub-normal levels) of a non- or sub-functional variant of Factor I. (Such a subject may carry one or more copies of an AMD-associated SNP, for example the subject may be homo- or heterozygous for one of the rare Factor I variants discussed further below.) Thus, the subject may have a low concentration (e.g. a lower than normal concentration) of Factor I in the eye and/or serum. For a human subject, the normal level of Factor I activity (C3b-inactivating and iC3b-degradation activity) may be equivalent to that provided by 30-40 µg/mL Factor I in the serum of the subject. Thus, in a subject with low Factor I activity, the Factor I activity in the serum may correspond to less than 30 µg/mL and greater than 0 µg/mL Factor I, such as 0-20 or 0-10 µg/mL (these being ranges of Factor I serum concentration which may encompass a subject having a low Factor I concentration).

Thus, the subject to be treated by the present invention may suffer from a complement-mediated disorder of the eye such as AMD, more particularly dry AMD (e.g. characterised by geographic atrophy), or may be at risk of developing such a disorder. For example, the subject may be homozygous or heterozygous susceptible for one or more SNPs associated with the complement-mediated disorder.

In one embodiment, the subject is at risk of developing AMD. For example, the subject may be homozygous or heterozygous susceptible for one or more SNPs associated with AMD, for example rare mutations in Factor I associated with advanced AMD which commonly result in reduced serum Factor I levels (Kavanagh et al., Hum Mol Genet. 2015 Jul 1;24(13):3861-70). In particular the subject may carry one or two copies of one or more of the following rare Factor I variants: rs144082872 (encoding P50A); 4:110687847 (encoding P64L); rs141853578 (encoding G119R); 4:110685721 (encoding V152M); 4:110682846 (encoding G162D); 4:110682801 (encoding N177I); rs146444258 (encoding A240G); rs182078921 (encoding G287R); rs41278047 (encoding K441R); rs121964913 (encoding R474).

Methods described herein may further comprise determining whether the subject is at risk of developing a complement-mediated disorder (for example AMD), for example by determining whether the subject is homozygous or heterozygous susceptible for one or more SNPs associated with the complement-mediated disorder (for example, by determining whether the subject is homozygous or heterozygous susceptible for one or more of the rare Factor I variants associated with AMD listed above).

Alternatively, the subject may have a normal level of endogenous Factor I activity or concentration, for example in the eye and/or serum and/or may not carry a rare variant Factor I allele.

In one embodiment, administration of the AAV vector, isolated cell or pharmaceutical composition of the invention thereby increases the level of C3b-inactivating and iC3b-degradation activity in the eye of the subject. In another embodiment, administration of the AAV vector, isolated cell or pharmaceutical composition of the invention thereby increases the level of C3b-inactivating and iC3b-degradation activity in the eye of the subject to a level that exceeds a normal level in the eye. More particularly, the level of C3b-inactivating and iC3b-degradation activity is increased in the RPE of the eye.

It will be appreciated that the C3b-inactivating and iC3b-degradation activity in the subject following expression of the Factor I or fragment thereof from the AAV vector of the invention may comprise C3b-inactivating and iC3b-degradation activity from the subject's endogenous Factor I (i.e. the subject's Factor I not produced by expression from the AAV vector), and C3b-inactivating and iC3b-degradation activity produced by expression from the AAV vector of the invention, such that the total level of C3b-inactivating and iC3b-degradation activity in the subject exceeds a normal level.

In one embodiment, the level of C3b-inactivating and iC3b-degradation activity in the subject, for example in the eye, is increased to a level that is at least 5%, 10%, 15%, 20% or 25% above the normal level.

In another embodiment, the level of C3b-inactivating and iC3b-degradation activity in the subject, for example in the eye, is increased to a level that is up to twice the normal level, or up to 80%, 60%, 40% or 20% above the normal level.

For example, the level of C3b-inactivating and iC3b-degradation activity in the subject, for example in the eye, may be increased to a level that is 5-100%, 5-80%, 5-60%, 5-40%, 5-20%, 10-100%, 10-80%, 10-60%, 10-40%, 10-20%, 15-100%, 15-80%, 15-60%, 15-40%, 15-20%, 20-100%, 20-80%, 20-60%, 20-40%, 25-100%, 25-80%, 25-60% or 25-40% above the normal level.

In one embodiment, administration of the AAV vector, isolated cell or pharmaceutical composition of the invention does not detectably increase the level of C3b-inactivating and iC3b-degradation activity in the plasma/serum of the subject. In another embodiment, administration of the AAV vector, isolated cell or pharmaceutical composition of the invention does not detectably increase the level of C3b-inactivating and iC3b-degradation activity in the plasma/serum of the subject to a level greater than the normal level.

Also described herein, prior to administration of an AAV vector, isolated cell or pharmaceutical composition of the disclosure, the subject may have low levels (e.g. lower than normal levels) of Factor H, for example low levels of Factor H in the eye and/or low serum levels of Factor H. For a human subject, the normal level of Factor H may be about 200-500 µg/mL in the serum of the subject. Thus, in a subject with low levels of Factor H, the levels in the serum may be less than 200 µg/mL and greater than 0 µg/mL, such as 0-100 µg/mL. Alternatively, the subject may have a normal level of endogenous Factor H, for example in the eye and/or serum.

### Factor I

Complement factor I (Factor I, CFI), also known as C3b/C4b inactivator, is a protein that in humans is encoded by the *CFI* gene.

Factor I is a serine protease that circulates in a zymogen-like state (Roversi et al.; PNAS; 2011; 108(31):12839-12844) at a concentration of ~35 µg/mL (Nilsson et al; Mol Immunol 2011, 48(14):1611-1620). The Factor I protein is a heavily N-glycosylated heterodimer consisting of two polypeptide chains linked by a single disulfide bond. The heavy chain (50 kDa) comprises an N-terminal region; an FI membrane attack complex (FIMAC) domain; a CD5 like-domain or scavenger receptor cysteine-rich (SRCR) domain; two low-density lipoprotein receptor (LDLr) domains; and a C-terminal region of unknown function that is a site of sequence variability across species (Roversi *et al.;* as above). The light chain (38 kDa) contains the serine protease (SP) domain with the conserved catalytic residues (Goldberger et al; J Biol Chem 1987, 262(21):10065-10071).

Factor I inactivates C3b by cleaving it into iC3b, C3d and C3d,g and, in an analogous way, C4b into C4c and C4d. To properly perform its functions, Factor I requires the presence of cofactor proteins such as C4b-Binding Protein (C4BP), Complement Factor H (CFH), Complement Receptor 1 (CR1/CD35) and Membrane Cofactor Protein (MCP/CD46) (Degn et al.; Am J Hum Genet 2011, 88(6):689-705).

iC3b is incapable of associating with factor B, and thus cannot perpetuate amplification of the complement cascade or activation through the alternative pathway. Hence, once C3b has been cleaved to iC3b, neither alternative pathway initiation nor terminal complement cascade activation occurs.

iC3b is capable of providing a proinflammatory action by binding to, and activating, complement receptor 3 (CR3)(CD11b/CD18) on polymorphonuclear leukocytes (mostly neutrophils), NK cells, and mononuclear phagocytes such as macrophages.

Factor I is capable of processing iC3b into C3d,g via a protease activity requiring the cofactor, CR1. C3d,g is unable to bind to CR3. Since iC3b reacting with the complement receptor CR3 is a major mechanism by which complement activation gives rise to inflammation, the breakdown of iC3b to C3d,g is essential for reducing complement-induced inflammation (Lachmann (2009), Adv. Immunol., 104:115-149).

Factor I's unique ability to both promote cleavage of C3b to iC3b as well as accelerate breakdown of iC3b - combined with its relatively low concentration in human serum, with implications for the amount required to be delivered for therapeutic efficacy - make it a particularly advantageous target.

In one embodiment a Factor I polypeptide or a fragment thereof is capable of cleaving C3b into an inactive degradation product. For example, the Factor I polypeptide or fragment thereof may be capable of cleaving C3b into iC3b.

In one embodiment a Factor I polypeptide or a fragment thereof is capable of processing iC3b into an inactive degradation product. For example, the Factor I polypeptide or fragment thereof may be capable of processing iC3b into C3d,g.

In a preferred embodiment the Factor I polypeptide or a fragment thereof is capable of cleaving C3b into iC3b and processing iC3b into C3d,g.

The fragment of Factor I may retain at least 50%, 60%, 70%, 80%, 90%, 95% or 100% of the C3b-inactivating and iC3b-degradation activity of native Factor I. The C3b-inactivating and iC3b-degradation activity of the fragment of Factor I, and native Factor I, may be determined using any suitable method known to those of skill in the art. For example, measurement of Factor I proteolytic activity is described in Hsiung et al. (Biochem. J. (1982) 203, 293-298). Both haemolytic and conglutinating assays for FI activity are described in Lachmann PJ & Hobart MJ (1978) "Complement Technology" in Handbook of Experimental Immunology 3rd edition Ed DM Weir Blackwells Scientific Publications Chapter 5A p17. A more detailed description, also including a proteolytic assay, is given by Harrison RA(1996) in "Weir's Handbook of Experimental Immunology" 5th Edition Eds; Herzenberg Leonore A'Weir DM, Herzenberg Leonard A & Blackwell C Blackwells Scientific Publications Chapter 75 36-37. The conglutinating assay is highly sensitive and can be used for detecting both the first (double) clip converting fixed C3b to iC3b and acquiring reactivity with conglutinin; and for detecting the final clip to C3dg by starting with fixed iC3b and looking for the loss of reactivity with conglutinin. The haemolytic assay is used for the conversion of C3b to iC3b, and the proteolytic assay detects all the clips.

In one embodiment, the Factor I is human Factor I.

An example human Factor I protein is the human Factor I protein having the UniProtKB accession number P05156. This exemplified sequence is 583 amino acids in length (shown as SEQ ID NO: 1) of which amino acids 1 to 18 form a signal sequence.

In one embodiment, the amino acid sequence of Factor I is the sequence shown as SEQ ID NO: 1. In one embodiment, the amino acid sequence of Factor I is the sequence shown as positions 19 to 583 of SEQ ID NO: 1.

MKLLHVFLLF LCFHLRFCKV TYTSQEDLVE KKCLAKKYTH LSCDKVFCQP
WQRCIEGTCV CKLPYQCPKN GTAVCATNRR SFPTYCQQKS LECLHPGTKF
LNNGTCTAEG KFSVSLKHGN TDSEGIVEVK LVDQDKTMFI CKSSWSMREA
NVACLDLGFQ QGADTQRRFK LSDLSINSTE CLHVHCRGLE TSLAECTFTK
RRTMGYQDFA DVVCYTQKAD SPMDDFFQCV NGKYISQMKA CDGINDCGDQ
SDELCCKACQ GKGFHCKSGV CIPSQYQCNG EVDCITGEDE VGCAGFASVT
QEETEILTAD MDAERRRIKS LLPKLSCGVK NRMHIRRKRI VGGKRAQLGD
LPWQVAIKDA SGITCGGIYI GGCWILTAAH CLRASKTHRY QIWTTVVDWI

HPDLKRIVIE YVDRIIFHEN YNAGTYQNDI ALIEMKKDGN KKDCELPRSI
PACVPWSPYL FQPNDTCIVS GWGREKDNER VFSLQWGEVK LISNCSKFYG
NRFYEKEMEC AGTYDGSIDA CKGDSGGPLV CMDANNVTYV WGVVSWGENC
GKPEFPGVYT KVANYFDWIS YHVGRPFISQ YNV

(SEQ ID NO: 1)

In one embodiment, the amino acid sequence of Factor I is the sequence shown as SEQ ID NO: 9, which corresponds to NCBI Accession No. NP_000195. In one embodiment, the amino acid sequence of Factor I is the sequence shown as positions 19 to 583 of SEQ ID NO: 9.

MKLLHVFLLF LCFHLRFCKV TYTSQEDLVE KKCLAKKYTH LSCDKVFCQP WQRCIEGTCV
CKLPYQCPKN GTAVCATNRR SFPTYCQQKS LECLHPGTKF LNNGTCTAEG KFSVSLKHGN
TDSEGIVEVK LVDQDKTMFI CKSSWSMREA NVACLDLGFQ QGADTQRRFK LSDLSINSTE
CLHVHCRGLE TSLAECTFTK RRTMGYQDFA DVVCYTQKAD SPMDDFFQCV NGKYISQMKA
CDGINDCGDQ SDELCCKACQ GKGFHCKSGV CIPSQYQCNG EVDCITGEDE VGCAGFASVA
QEETEILTAD MDAERRRIKS LLPKLSCGVK NRMHIRRKRI VGGKRAQLGD LPWQVAIKDA
SGITCGGIYI GGCWILTAAH CLRASKTHRY QIWTTVVDWI HPDLKRIVIE YVDRIIFHEN
YNAGTYQNDI ALIEMKKDGN KKDCELPRSI PACVPWSPYL FQPNDTCIVS GWGREKDNER
VFSLQWGEVK LISNCSKFYG NRFYEKEMEC AGTYDGSIDA CKGDSGGPLV CMDANNVTYV
WGVVSWGENC GKPEFPGVYT KVANYFDWIS YHVGRPFISQ YNV

(SEQ ID NO: 9)

An example of a nucleotide sequence encoding Factor I is the nucleotide sequence having the NCBI Accession No. NM_000204. In one embodiment, the nucleotide sequence encoding Factor I is the nucleotide sequence having the NCBI Accession No. NM_000204.

In one embodiment, the nucleotide sequence encoding Factor I is the nucleotide sequence shown as SEQ ID NO: 2.

atgaagcttc ttcatgtttt cctgttattt ctgtgcttcc acttaaggtt ttgcaaggtc
acttatacat ctcaagagga tctggtggag aaaaagtcct tagcaaaaaa atatactcac
ctctcctgcg ataaagtctt ctgccagcca tggcagacat gcattgaggg cacctgtgtt
tgtaaactac cctatcagtg cccaaagaat ggcactgcag tgtgtgcaac taacaggaga
agcttcccaa catactgtca acaaaagagt ttggaatctc ttcatccagg gacaaagttt
ttaaataacg gaacatgcac agccgaagga aagtttactg tttccttgaa gcatggaaat
acagattcag acggaatagt tgaagtaaaa cttgtggacc aagataagac aatgttcata
tgcaaaagca gctggagcat gagggaagcc aacgtggcct gccttgacct tgggtttcaa
caaggtgctg atactcaaag aaggtttaag ttgtctgatc tctctataaa ttccactgaa
tgtctacatg tgcattgccg aggattagag accagtttgg ctgaatgtac ttttactaag
agaagaacta tcggttacca ggatttcgct gatgtggttt gttatacaca gaaagcagat
tctccaatgg atgacttctt tcagtgtgtg aatgggaaat acatttctca gatgaaagcc
tgtgatggta tcaatgattg tggagaccaa agtgatgaac tgtgttgtaa agcatgccaa
ggcaaaggct tccattgcaa atcgggtgtt tgcattccaa gccagtatca atgcaatggt
gaggtggact gcattacagg ggaagatgaa gttggctctg caggctttgc atctgtggct
caagaagaaa cagaaatttt gactgctgac atggatgcag aaagaagacg gataaaatca
ttattaccta aactatcttg tggagttaaa aacagaatgc acattcgaag gaaacgaatt
gtgggaggaa agcgagcaca actgggagac ctcccatggc aggtggcaat taaggatgcc
agtggaatca cctgtggggg aatttatatt ggtggctgtt ggattctgac tgctgcacat
tgtctcagag ccagtaaaac tcatcgttac caaatatgga caacagtagt agactggata
caccccgacc ttaaacgtat agtaattgaa tacgtggata gaattatttt ccatgaaaac
tacaatgcag gcacttacca aaatgacatc gctttgattg aaatgaaaaa agacggaaac
aaaaaagatt gtgagctgcc tcgttccatc cctgcctgtg tcccctggtc tccttaccta
ttccaaccta atgatacatg catcgtttct ggctgggcac gagaaaaaga taacgaaaga
gtcttttcac ttcagtgggg tgaagttaaa ctaataacca actgctctaa gttttacgga
aatcgtttct atgaaaaaga aatggaatgt gcaggtacat atgatggttc catcgatgcc

tgtaaagggg actctggagg ccccttagtc tgtatggatg ccaacaatgt gacttatgtc
tggggtgttg tgagttgggg ggaaaactgt ggaaaaccag agttcccagg tgtttacacc
aaagtggcca attattttga ctggattagc taccatgtag gaaggccttt tatttctcag
tacaatgtat aa

(SEQ ID NO: 2)

The nucleotide sequences used in the invention may be codon-optimised. Codon optimisation has previously been described in WO 1999/041397 and WO 2001/079518. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available.

In one embodiment, the nucleotide sequence encoding Factor I is the nucleotide sequence shown as SEQ ID NO: 8.

ATGAAGCTGCTGCATGTCTTTCTGCTGTTTCTGTGCTTCCATCTGCGGTTCTGTAAAGTGACCTATACTAGC
CAGGAGGATCTGGTGGAGAAGAAGTGTCTGGCCAAGAAGTACACACACCTGAGCTGCGACAAGGTGTTCTGT
CAGCCTTGGCAGCGGTGCATCGAGGGCACCTGCGTGTGCAAGCTGCCTTACCAGTGCCCAAAGAACGGCACC
GCCGTGTGCGCCACAAATCGGAGATCTTTTCCAACATATTGCCAGCAGAAGAGCCTGGAGTGTCTGCACCCC
GGCACCAAGTTCCTGAACAATGGCACCTGCACAGCCGAGGGCAAGTTTTCTGTGAGCCTGAAGCACGGCAAC
ACAGATAGCGAGGGCATCGTGGAGGTGAAGCTGGTGGACCAGGATAAGACCATGTTCATCTGTAAGAGCTCC
TGGTCCATGAGGGAGGCAAACGTGGCATGCCTGGATCTGGGATTCCAGCAGGGAGCAGACACACAGAGGCGC
TTTAAGCTGTCCGACCTGTCTATCAATAGCACCGAGTGCCTGCACGTGCACTGTAGGGGCCTGGAGACATCC
CTGGCAGAGTGCACCTTCACAAAGCGGAGAACCATGGGCTACCAGGACTTTGCCGACGTGGTGTGCTATACC
CAGAAGGCCGATAGCCCCATGGACGATTTCTTTCAGTGCGTGAACGGCAAGTATATCTCCCAGATGAAGGCC
TGCGACGGCATCAATGACTGTGGCGATCAGTCTGACGAGCTGTGCTGTAAGGCCTGTCAGGGCAAGGGCTTC
CACTGCAAGAGCGGCGTGTGCATCCCTTCCCAGTACCAGTGCAACGGCGAGGTGGATTGTATCACAGGAGAG
GACGAAGTGGGATGCGCAGGATTTGCATCTGTGGCACAGGAGGAGACAGAGATCCTGACAGCCGACATGGAT
GCCGAGAGGCGCCGGATCAAGTCTCTGCTGCCTAAGCTGAGCTGTGGCGTGAAGAATCGGATGCACATCAGA
AGGAAGCGCATCGTGGGAGGCAAGAGGGCACAGCTGGGCGATCTGCCATGGCAGGTGGCCATCAAGGACGCC
TCTGGCATCACCTGCGGCGGCATCTACATCGGAGGATGTTGGATCCTGACCGCAGCACACTGCCTGAGAGCA
AGCAAGACACACAGGTATCAGATCTGGACCACAGTGGTGGATTGGATCCACCCAGACCTGAAGAGAATCGTG
ATCGAGTACGTGGATAGGATCATCTTTCACGAGAACTACAATGCCGGCACATATCAGAACGACATCGCCCTG
ATCGAGATGAAGAAGGATGGCAATAAGAAGGACTGTGAGCTGCCCAGATCCATCCCTGCATGCGTGCCATGG
AGCCCCTATCTGTTCCAGCCCAACGATACCTGCATCGTGTCCGGATGGGGAAGGGAGAAGGACAATGAGCGG
GTGTTTTCTCTGCAGTGGGGCGAGGTGAAGCTGATCTCCAACTGTTCTAAGTTCTACGGCAATAGGTTTTAT
GAGAAGGAGATGGAGTGCGCCGGCACCTACGATGGCAGCATCGACGCCTGTAAGGGCGATTCCGGAGGACCA
CTGGTGTGCATGGACGCAAACAATGTGACATACGTGTGGGGAGTGGTGTCCTGGGGAGAGAACTGCGGCAAG
CCAGAGTTCCCCGGCGTATATACCAAGGTGGCCAATTATTTTGATTGGATTTCCTACCACGTCGGCAGGCCC
TTTATTTCCCAGTATAATGTCTAA

(SEQ ID NO: 8)

The nucleotide sequence encoding Factor I or a fragment thereof may, for example, comprise a nucleotide sequence that has at least 70%, 80%, 90%, 95%, 96%, 97%, 98% 99% or 100% identity to SEQ ID NO: 2 or 8, wherein the protein encoded by the nucleotide sequence substantially retains a functional activity of the protein represented by SEQ ID NO: 1 or 9.

The nucleotide sequence encoding Factor I or a fragment thereof may, for example, comprise a nucleotide sequence that has at least 70%, 80%, 90%, 95%, 96%, 97%, 98% 99% or 100% identity to the sequence shown as positions 55 to 1752 of SEQ ID NO: 2 or 8, wherein the protein encoded by the nucleotide sequence substantially retains a functional activity of the protein represented by SEQ ID NO: 1 or 9.

The nucleotide sequence encoding Factor I or a fragment thereof may, for example, encode an amino acid sequence that has at least 70%, 80%, 90%, 95%, 96%, 97%, 98% 99% or 100% identity to SEQ ID NO: 1 or 9, wherein the amino acid sequence substantially retains a functional activity of the protein represented by SEQ ID NO: 1 or 9.

The nucleotide sequence encoding Factor I or a fragment thereof may, for example, encode an amino acid sequence that has at least 70%, 80%, 90%, 95%, 96%, 97%, 98% 99% or 100% identity to the sequence shown as positions 19 to 583 of SEQ ID NO: 1 or 9, wherein the amino acid sequence substantially retains a functional activity of the protein represented by SEQ ID NO: 1 or 9.

An advantage of the invention is that Factor I is particularly difficult to prepare in the form of a purified protein. Accordingly, the inventors have devised a way of modulating the complement system, for example to enable treatments of age-related macular degeneration (AMD), by administering Factor I in the form of an AAV vector comprising a Factor I-encoding nucleotide sequence. The AAV vector may be administered to a site of interest, for example the eye, to enable in situ translation of the Factor I polypeptide.

### Factor H

Complement factor H (Factor H, CFH) is a complement control protein.

It is a large (155 kDa), soluble glycoprotein that is present in human plasma at typical concentration of 200-300 µg/mL (Hakobyan *et al.;* 2008; 49(5): 1983-90). The principal function of Factor H is to regulate the alternative pathway of the complement system.

Factor H provides cofactor activity for the Factor I-mediated cleavage of C3b. Factor H also increases the rate of dissociation of the C3bBb complex (C3 convertase) and the (C3b)NBB complex (C5 convertase) and thereby reduces the activity of the alternative complement pathway.

Factor H is made up of 20 complement control protein (CCP) modules (also referred to as Short Consensus Repeats or sushi domains) connected to one another by short linkers (of between three and eight amino acid residues) and arranged in an extended head to tail fashion. Each of the CCP modules consists of around 60 amino acids with four cysteine residues disulfide bonded in a 1-3 2-4 arrangement, and a hydrophobic core built around an almost invariant tryptophan residue. The CCP modules are numbered from 1-20 (from the N-terminus of the protein). CCPs 1-4 and CCPs 19-20 engage with C3b while CCPs 7 and CCPs 19-20 bind to GAGs and sialic acid (Schmidt et al; 2008; Journal of Immunology 181 (4): 2610-9).

It has been shown that gene therapy using Factor H can ameliorate induced AMD-like pathology in mice (Cashman et al. (2015) J. Gene Med. 17: 229-243). Mice were co-injected subretinally with: (i) an adenoviral vector expressing complement component C3, which had previously been shown to recapitulate many pathological features of human AMD; and (ii) an adenoviral vector expressing Factor H. Relative to control animals receiving GFP instead of Factor H, the Factor H-transduced mice showed 91% reduction in endothelial cell proliferation and 69% attenuation of RPE atrophy. Electroretinography showed improved retinal function in mice receiving Factor H, and immunocytochemistry of rhodopsin and RPE65 was consistent with the rescue of photoreceptors and RPE in such animals.

In one embodiment a Factor H polypeptide or a fragment or derivative thereof is capable of acting as a cofactor for the Factor I-mediated cleavage of C3b. In one embodiment a Factor H polypeptide or a fragment or derivative thereof is capable of increasing the rate of dissociation of C3 convertase and C5 convertase.

In a preferred embodiment a Factor H polypeptide or a fragment or derivative thereof is capable of acting as a cofactor for the Factor I-mediated cleavage of C3b and increasing the rate of dissociation of C3 convertase and C5 convertase.

In one embodiment, the Factor H is human Factor H.

An example human Factor H protein is the human Factor H protein having the UniProtKB accession number P08603. This exemplified sequence is 1231 amino acids in length (shown as SEQ ID NO: 3) of which amino acids 1 to 18 form a signal sequence.

In one embodiment, the amino acid sequence of Factor H is the sequence shown as SEQ ID NO: 3. In one embodiment, the amino acid sequence of Factor H is the sequence shown as positions 19 to 1231 of SEQ ID NO: 3.

MRLLAKIICL MLWAICVAED CNELPPRRNT EILTGSWSDQ TYPEGTQAIY
KCRPGYRSLG NVIMVCRKGE WVALNPLRKC QKRPCGHPGD TPFGTFTLTG
GNVFEYGVKA VYTCNEGYQL LGEINYRECD TDGWTNDIPI CEVVKCLPVT
APENGKIVSS AMEPDREYHF GQAVRFVCNS GYKIEGDEEM HCSDDGFWSK
EKPKCVEISC KSPDVINGSP ISQKIIYKEN ERFQYKCNMG YEYSERGDAV
CTESGWRPLP SCEEKSCDNP YIPNGDYSPL RIKHRTGDEI TYQCRNGFYP
ATRGNTAKCT STGWIPAPRC TLKPCDYPDI KHGGLYHENM RRPYFPVAVG
KYYSYYCDEH FETPSGSYWD HIHCTQDGWS PAVPCLRKCY FPYLENGYNQ
NYGRKFVQGK SIDVACHPGY ALPKAQTTVT CMENGWSPTP RCIRVKTCSK
SSIDIENGFI SESQYTYALK EKAKYQCKLG YVTADGETSG SITCGKDGWS
AQPTCIKSCD IPVFMNARTK NDFTWFKLND TLDYECHDGY ESNTGSTTGS
IVCGYNGWSD LPICYERECE LPKIDVHLVP DRKKDQYKVG EVLKFSCKPG
FTIVGPNSVQ CYHFGLSPDL PICKEQVQSC GPPPELLNGN VKEKTKEEYG
HSEVVEYYCN PRFLMKGPNK IQCVDGEWTT LPVCIVEEST CGDIPELEHG
WAQLSSPPYY YGDSVEFNCS ESFTMIGHRS ITCIHGVWTQ LPQCVAIDKL
KKCKSSNLII LEEHLKNKKE FDHNSNIRYR CRGKEGWIHT VCINGRWDPE
VNCSMAQIQL CPPPPQIPNS HNMTTTLNYR DGEKVSVLCQ ENYLIQEGEE
ITCKDGRWQS IPLCVEKIPC SQPPQIEHGT INSSRSSQES YAHGTKLSYT
CEGGFRISEE NETTCYMGKW SSPPQCEGLP CKSPPEISHG VVAHMSDSYQ
YGEEVTYKCF EGFGIDGPAI AKCLGEKWSH PPSCIKTDCL SLPSFENAIP
MGEKKDVYKA GEQVTYTCAT YYKMDGASNV TCINSRWTGR PTCRDTSCVN
PPTVQNAYIV SRQMSKYPSG ERVRYQCRSP YEMFGDEEVM CLNGNWTEPP
QCKDSTGKCG PPPPIDNGDI TSFPLSVYAP ASSVEYQCQN LYQLEGNKRI
TCRNGQWSEP PKCLHPCVIS REIMENYNIA LRWTAKQKLY SRTGESVEFV
CKRGYRLSSR SHTLRTTCWD GKLEYPTCAK R

(SEQ ID NO: 3)

An example of a nucleotide sequence encoding Factor H is the nucleotide sequence having the NCBI Accession No. NM_000186. In one embodiment, the nucleotide sequence encoding Factor H is the nucleotide sequence having the NCBI Accession No. NM_000186.

In one embodiment, the nucleotide sequence encoding Factor H is the nucleotide sequence shown as SEQ ID NO: 4.

atgagacttc tagcaaagat tatttgcctt atgttatcgg ctatttgtgt agcagaagat
tgcaatgaac ttcctccaag aagaaataca gaaattctga caggttcctg gtctgaccaa
acatatccag aaggcaccca ggctatctat aaatgccgcc ctggatatag atctcttgga
aatgtaataa tcgtatgcag gaagggagaa tgggttgctc ttaatccatt aaggaaatgt
cagaaaaggc cctgtggaca tcctggagat actccttttg gtacttttac ccttacagga
ggaaatgtgt ttgaatatgg tgtaaaagct gtgtatacat gtaatgaggg gtatcaattg
ctaggtgaga ttaattaccg tgaatgtgac acagatggat ggaccaatga tattcctata
tgtgaagttg tcaagtgttt accagtgaca gcaccagaga atggaaaaat tgtcagtagt
gcaatggaac cagatcggga ataccatttt ggacaagcag tacggtttgt atgtaactca
ggctacaaga ttgaaggaga tgaagaaatg cattgttcag acgatggttt ttggagtaaa
gagaaaccaa agtgtgtgga aatttcatgc aaatccccag atgttataaa tggatctcct
atatctcaga agattattta taaggagaat gaacgatttc aatataaatg taacatgggt
tatgaataca gtgaaagagg agatgctgta tgcactgaat ctggatggcg tccgttgcct
tcatgtgaag aaaaatcatg tgataatcct tatattccaa atggtgacta ctcaccttta
aggattaaac acagaactgg agatgaaatc acgtaccagt gtagaaatgg tttttatcct
gcaacccggg gaaatacagc aaaatgcaca agtactggct ggatacctgc tccgagatgt
accttgaaac cttgtgatta tccagacatt aaacatggag gtctatatca tgagaatatg
cgtagaccat actttccagt agctgtagga aaatattact cctattactg tgatgaacat
tttgagactc cgtcaggaag ttactgggat cacattcatt gcacacaaga tggatggtcg
ccagcagtac catgcctcag aaaatgttat tttccttatt tggaaaatgg atataatcaa
aatcatggaa gaaagtttgt acagggtaaa tctatagacg ttgcctgcca tcctggctac
gctcttccaa aagcgcagac cacagttaca tgtatggaga atggctggtc tcctactccc
agatgcatcc gtgtcaaaac atgttccaaa tcaagtatag atattgagaa tgggtttatt
tctgaatctc agtatacata tgccttaaaa gaaaaagcga aatatcaatg caaactagga
tatgtaacag cagatggtga aacatcagga tcaattacat gtgggaaaga tggatggtca
gctcaaccca cgtgcattaa atcttgtgat atcccagtat ttatgaatgc cagaactaaa

aatgacttca catggtttaa gctgaatgac acattggact atgaatgcca tgatggttat
gaaagcaata ctggaagcac cactggttcc atagtgtctg gttacaatgg ttggtctgat
ttacccatat gttatgaaag agaatgcgaa cttcctaaaa tagatgtaca cttagttcct
gatcgcaaga aagaccagta taaagttgga gaggtgttga aattctcctg caaaccagga
tttacaatag ttggacctaa ttccgttcag tgctaccact ttggattgtc tcctgacctc
ccaatatgta aagagcaagt acaatcatgt ggtccacctc ctgaactcct caatgggaat
gttaaggaaa aaacgaaaga agaatatgga cacagtgaag tggtggaata ttattgcaat
cctagatttc taatgaaggg acctaataaa attcaatgtg ttgatggaga gtggacaact
ttaccagtgt gtattgtgga ggagagtacc tgtggagata tacctgaact tgaacatggc
tgggcccagc tttcttcccc tccttattac tatggagatt cagtggaatt caattgctca
gaatcattta caatgattgg acacagatca attacgtgta ttcatggagt atggacccaa
cttccccagt gtgtggcaat agataaactt aagaagtgca aatcatcaaa tttaattata
cttgaggaac atttaaaaaa caagaaggaa ttcgatcata attctaacat aaggtacaga
tgtagaggaa aagaaggatg gatacacaca gtctgcataa atggaagatg ggatccagaa
gtgaactgct caatggcaca aatacaatta tgcccacctc cacctcagat tcccaattct
cacaatatga caaccacact gaattatcgg gatggagaaa aagtatctgt tctttgccaa
gaaaattatc taattcagga aggagaagaa attacatgca aagatggaag atggcagtca
ataccactct gtgttgaaaa aattccatgt tcacaaccac ctcagataga acacggaacc
attaattcat ccaggtcttc acaagaaagt tatgcacatg ggactaaatt gagttatact
tgtgagggtg gtttcaggat atctgaagaa aatgaaacaa catgctacat gggaaaatgg
agttctccac ctcagtgtga aggccttcct tgtaaatctc cacctgagat ttctcatggt
gttgtagctc acatgtcaga cagttatcag tatggagaag aagttacgta caaatgtttt
gaaggttttg gaattgatgg gcctgcaatt gcaaaatgct taggagaaaa atggtctcac
cctccatcat gcataaaaac agattgtctc agtttaccta gctttgaaaa tgccataccc
atgggagaga agaaggatgt gtataaggcg ggtgagcaag tgacttacac ttgtgcaaca
tattacaaaa tggatggagc cagtaatgta acatgcatta atagcagatg gacaggaagg
ccaacatgca gagacacctc ctgtgtgaat ccgcccacag tacaaaatgc ttatatagtg
tcgagacaga tgagtaaata tccatctggt gagagagtac gttatcaatg taggagccct
tatgaaatgt ttggggatga agaagtgatg tgtttaaatg gaaactggac ggaaccacct
caatgcaaag attctacagg aaaatgtggg ccccctccac ctattgacaa tggggacatt
acttcattcc cgttgtcagt atatgctcca gcttcatcag ttgagtacca atgccagaac
ttgtatcaac ttgagggtaa caagcgaata acatgtagaa atggacaatg gtcagaacca
ccaaaatgct tacatccgtg tgtaatatcc cgagaaatta tggaaaatta taacatagca
ttaaggtgga cagccaaaca gaagctttat tcgagaacag gtgaatcagt tgaatttgtg
tgtaaacggg gatatcgtct ttcatcacgt tctcacacat tgcgaacaac atgttgggat
gggaaactgg actatccaac ttgtgcaaaa agatag

(SEQ ID NO: 4)

The nucleotide sequence encoding Factor H or a fragment or derivative thereof may, for example, comprise a nucleotide sequence that has at least 70%, 80%, 90%, 95%, 96%, 97%, 98% 99% or 100% identity to SEQ ID NO: 4, wherein the protein encoded by the nucleotide sequence substantially retains a functional activity of the protein represented by SEQ ID NO: 3.

The nucleotide sequence encoding Factor H or a fragment or derivative thereof may, for example, comprise a nucleotide sequence that has at least 70%, 80%, 90%, 95%, 96%, 97%, 98% 99% or 100% identity to the sequence shown as positions 55 to 3696 of SEQ ID NO: 4, wherein the protein encoded by the nucleotide sequence substantially retains a functional activity of the protein represented by SEQ ID NO: 3.

The nucleotide sequence encoding Factor H of the present invention may, for example, encode an amino acid sequence that has at least 70%, 80%, 90%, 95%, 96%, 97%, 98% 99% or 100% identity to SEQ ID NO: 3, wherein the amino acid sequence substantially retains a functional activity of the protein represented by SEQ ID NO: 3.

The nucleotide sequence encoding Factor H of the present invention may, for example, encode an amino acid sequence that has at least 70%, 80%, 90%, 95%, 96%, 97%, 98% 99% or 100% identity to the sequence shown as positions 19 to 1231 of SEQ ID NO: 3, wherein the amino acid sequence substantially retains a functional activity of the protein represented by SEQ ID NO: 3.

### Factor D

Complement factor D (Factor D, CFD) is involved in the alternative complement pathway of the complement system. It functions to cleave factor B to factor Bb and Ba.

Factor D is a member of the trypsin family of peptidases. All members of the chymotrypsin family of serine proteases have very similar structures. In all cases, including factor D, there are two antiparallel β-barrel domains with each barrel containing six β-strands with the same typology in all enzymes. The major difference in backbone structure between Factor D and the other serine proteases of the chymotrypsin family is in the surface loops connecting the secondary structural elements.

The alternative complement activation cascade is initiated by the spontaneous hydrolysis of C3, which is abundant in the blood plasma. "Tickover" occurs through the spontaneous cleavage of the thioester bond in C3 to form C3(H₂O).

This change in shape allows the binding of plasma protein Factor B, which allows Factor D to cleave Factor B into Ba and Bb. Bb remains part of the C3(H₂O) to form C3(H₂O)Bb. This complex is also known as a fluid-phase C3-convertase. This convertase, although only produced in small amounts, can cleave multiple C3 proteins into C3a and C3b.

The AAV vector of the present invention may comprise a nucleotide sequence which encodes an anti-Factor D antibody.

The anti-factor D antibody may bind to factor D and reduce or prevent a functional activity of Factor D. For example, the anti-factor D antibody may reduce or prevent Factor D binding to Factor B and/or the Factor D cleavage of Factor B.

Suitable anti-Factor D antibodies are known in the art. Such antibodies include, but are not limited to lampalizumab.

### Complement component 5

Complement component 5 (C5) is the fifth component of complement, which plays an important role in inflammatory and cell killing processes. C5 is composed of alpha and beta polypeptide chains that are linked by a disulfide bridge.

C5 is cleaved by protease C5-convertase into Complement component 5a (C5a) and C5b fragments. C5b is important in late events of complement cascade, whereas C5a acts as highly inflammatory peptide. The origin of C5 is generally hepatocytes but its synthesis can also be found in macrophages and this may cause local increases of C5a. C5a has chemotactic and anaphylatoxic properties, it is essential in the innate immunity but it is also linked with the adaptive immunity. The increase production of C5a is connected with a number of inflammatory diseases.

C5a is an anaphylatoxin, causing increased expression of adhesion molecules on endothelium, contraction of smooth muscle, and increased vascular permeability. C5a des-Arg (which lacks the C-terminal arginine) is a much less potent anaphylatoxin. Both C5a and C5a des-Arg can trigger mast cell degranulation, releasing proinflammatory molecules histamine and TNF-α. C5a is also an effective chemoattractant, initiating accumulation of complement and phagocytic cells at sites of infection or recruitment of antigen-presenting cells to lymph nodes. C5a plays a key role in increasing migration and adherence of neutrophils and monocytes to vessel walls. White blood cells are activated by upregulation of integrin avidity, the lipoxygenase pathway and arachidonic acid metabolism. C5a also modulates the balance between activating versus inhibitory IgG Fc receptors on leukocytes, thereby enhancing the autoimmune response.

The AAV vector of the present invention may comprise a nucleotide sequence which encodes an anti-C5 antibody.

The anti-C5 antibody may bind to C5 and prevent cleavage of C5 to C5a and C5b.

Suitable anti-C5 antibodies are known in the art. Such antibodies include, but are not limited to, eculizumab.

### Antibody

An antibody, refers to any portion of an antibody or a fragment thereof which retains the ability to bind to the same antigen target as the parental antibody.

The antibody may be a chimeric antibody. Chimeric antibodies may be produced by transplanting antibody variable domains from one species (for example, a mouse) onto antibody constant domains from another species (for example a human).

The antibody may be a full-length, classical antibody. For example the antibody may be an IgG, IgM or IgA molecule.

The antibody may be a functional antibody fragment. Specific antibody fragments include, but are not limited to, (i) the Fab fragment consisting of VL, VH, CL and CH1 domains, (ii) the Fd fragment consisting of the VH and CH1 domains, (iii) the Fv fragment consisting of the VL and VH domains of a single antibody, (iv) the dAb fragment, which consists of a single variable domain, (v) isolated CDR regions, (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site, (viii) bispecific single chain Fv dimers, and (ix) "diabodies" or "triabodies", multivalent or multispecific fragments constructed by gene fusion. The antibody fragments may be modified. For example, the molecules may be stabilized by the incorporation of disulphide bridges linking the VH and VL domains.

The antibody described herein may be a multispecific antibody, and notably a bispecific antibody, also sometimes referred to as "diabodies". These are antibodies that bind to two (or more) different antigens. The antibody may be a minibody. Minibodies are minimized antibody-like proteins comprising a scFv joined to a CH3 domain. In some cases, the scFv can be joined to the Fc region, and may include some or all of the hinge region.

The antibody may be a domain antibody (also referred to as a single-domain antibody or nanobody). This is an antibody fragment containing a single monomeric single variable antibody domain. Examples of single-domain antibodies include, but are not limited to, VHH fragments originally found in camelids and VNAR fragments originally found in cartilaginous fishes. Single-domain antibodies may also be generated by splitting the dimeric variable domains from common IgG molecules into monomers.

The antibody may be a synthetic antibody (also referred to as an antibody mimetic). Antibody mimetics include, but are not limited to, Affibodies, DARPins, Anticalins, Avimers, Versabodies and Duocalins.

### Age-related macular degeneration (AMD)

The clinical progression of AMD is characterised in stages according to changes in the macula. The hallmark of early AMD is drusen, which are accumulations of extracellular debris underneath the retina and appear as yellow spots in the retina on clinical exam and on fundus photographs. Drusens are categorised by size as small (<63µm), medium (63-124 µm) and large (>124µm). They are also considered as hard or soft depending on the appearance of their margins on opthalmological examination. While hard drusens have clearly defined margins, soft ones have less defined and fluid margins. The Age-related Eye Disease Study (AREDS) fundus photographic severity scale is one of the main classification systems used for this condition.

AMD is classified into "dry" and "wet" (exudative, or neovascular) forms. Dry AMD is more common than wet AMD, but the dry form can progress to the wet form, and the two occur simultaneously in a significant number of cases. Dry AMD is typically characterized by progressive apoptosis of cells in the RPE layer, overlying photoreceptor cells, and frequently also the underlying cells in the choroidal capillary layer. Confluent areas of RPE cell death accompanied by overlying photoreceptor atrophy are referred to as geographic atrophy (GA). Patients with this form of AMD experience a slow and progressive deterioration in central vision.

Wet AMD is characterized by bleeding and/or leakage of fluid from abnormal vessels that have grown from the choroidal vessels (choriocapillaris) beneath the RPE and the macula, which can be responsible for sudden and disabling loss of vision. It has been estimated that much of the vision loss that patients experience is due to such choroidal neovascularization (CNV) and its secondary complications.

The treatment or prevention of AMD described herein may reduce or prevent the appearance of an AMD phenotype described above. Preferably, the treatment of AMD enables maintenance or improvement in visual function.

In one embodiment, the treatment or prevention of AMD results in a prevention of or reduction in the formation of geographic atrophy. In another embodiment, the treatment or prevention of AMD results in slowing the progression of geographic atrophy. For example, it results in an at least 10%, 20%, 30%, 40%, 50%, 60%, 70%, 80% or 90% reduction in the increase in GA area over the 12 months following administration to a treated eye of a subject, relative to an untreated eye over the same period. In another embodiment, the treatment or prevention of AMD results in the treatment of geographic atrophy, for example a reduction in the amount of geographic atrophy.

In one embodiment, the treatment or prevention of AMD results in a prevention of or reduction in the formation of drusen. In another embodiment, the treatment or prevention of AMD results in a reduction in existing drusen, for example a reduction in the size and/or number of existing drusen.

In one embodiment, the treatment or prevention of AMD results in a prevention of or reduction in complement deposition. In another embodiment, the treatment or prevention of AMD results in a reduction in existing complement deposition.

In one embodiment, the treatment or prevention of AMD results in an improvement in or restoration of vision or visual acuity. In another embodiment, the treatment or prevention of AMD mitigates the loss of vision or visual acuity.

In one embodiment, the treatment or prevention of AMD results in an improvement in or restoration of reading speed in a subject. In another embodiment, the treatment or prevention of AMD mitigates the reduction in reading speed in a subject.

In one embodiment, the treatment or prevention of AMD results in a reduction or prevention of loss of photoreceptors and/or the retinal pigment epithelium (RPE).

### Diabetic retinopathy

Diabetic retinopathy is a condition characterised by damage to the blood vessels of the retina, which is caused by the high blood sugar levels associated with diabetes. If left untreated, diabetic retinopathy can cause blindness.

Although subjects with mild diabetic retinopathy may have good vision, two types of diabetic retinopathy, namely diabetic macular oedema (DMO) and proliferative diabetic retinopathy (PDR) may threaten the sight of the subject.

Diabetic macular oedema is characterised by the leakage of fluid from the damaged blood vessels in the back of the eye. The leaked fluid accumulates in the macula, which leads to swelling and blurred vision. This can eventually give rise to poor central vision and an inability to read or drive. Side vision usually remains normal.

Proliferative diabetic retinopathy is characterised by the closure of retinal blood vessels, leading to the growth of abnormal, fragile blood vessels on the surface of the retina. This may result in permanent loss of vision due to bleeding into the eye, scarring and retinal detachment.

### Structure of the eye

The medicaments disclosed herein may be delivered to a mammalian, preferably human eye in relation to the treatment or prevention of age-related macular degeneration (AMD).

The person skilled in the treatment of diseases of the eye will have a detailed and thorough understanding of the structure of the eye. However, the following structures of particular relevance to the invention are described.

### Retina

The retina is the multi-layered membrane, which lines the inner posterior chamber of the eye and senses an image of the visual world which is communicated to the brain via the optic nerve. In order from the inside to the outside of the eye, the retina comprises the layers of the neurosensory retina and retinal pigment epithelium, with the choroid lying outside the retinal pigment epithelium.

### Neurosensory retina and photoreceptor cells

The neurosensory retina harbours the photoreceptor cells that directly sense light. It comprises the following layers: internal limiting membrane (ILM); nerve fibre layer; ganglion cell layer; inner plexiform layer; inner nuclear layer; outer plexiform layer; outer nuclear layer (nuclei of the photoreceptors); external limiting membrane (ELM); and photoreceptors (inner and outer segments of the rods and cones).

The skilled person will have a detailed understanding of photoreceptor cells. Briefly, photoreceptor cells are specialised neurons located in the retina that convert light into biological signals. Photoreceptor cells comprise rod and cone cells, which are distributed differently across the retina.

Rod cells are distributed mainly across the outer parts of the retina. They are highly sensitive and provide for vision at low light levels. There are on average about 125 million rod cells in a normal human retina.

Cone cells are found across the retina, but are particularly highly concentrated in the fovea, a pit in the neurosensory retina that is responsible for central high resolution vision. Cone cells are less sensitive than rod cells. There are on average about 6-7 million cone cells in a normal human retina.

### Retinal pigment epithelium

The retinal pigment epithelium (RPE) is a pigmented layer of cells located immediately to the outside of the neurosensory retina. The RPE performs a number of functions, including transport of nutrients and other substances to the photoreceptor cells, and absorption of scattered light to improve vision.

### Choroid

The choroid is the vascular layer situated between the RPE and the outer sclera of the eye. The vasculature of the choroid enables provision of oxygen and nutrients to the retina.

### Adeno-associated viral (AAV) Vectors

In one aspect, the invention provides an AAV vector comprising a nucleotide sequence encoding Factor I or a fragment thereof capable of cleaving C3b into iC3b, wherein the nucleotide sequence encoding Factor I or the fragment thereof is operably linked to a polyadenylation site.

Preferably, the AAV vector is in the form of an AAV particle.

Methods of preparing and modifying viral vectors and viral vector particles, such as those derived from AAV, are well known in the art.

The AAV vector may comprise an AAV genome or a fragment or derivative thereof.

An AAV genome is a polynucleotide sequence, which encodes functions needed for production of an AAV particle. These functions include those operating in the replication and packaging cycle of AAV in a host cell, including encapsidation of the AAV genome into an AAV particle. Naturally occurring AAVs are replication-deficient and rely on the provision of helper functions in trans for completion of a replication and packaging cycle. Accordingly, the AAV genome of the AAV vector of the invention is typically replication-deficient.

The AAV genome may be in single-stranded form, either positive or negative-sense, or alternatively in double-stranded form. The use of a double-stranded form allows bypass of the DNA replication step in the target cell and so can accelerate transgene expression.

The AAV genome may be from any naturally derived serotype, isolate or clade of AAV. Thus, the AAV genome may be the full genome of a naturally occurring AAV. As is known to the skilled person, AAVs occurring in nature may be classified according to various biological systems.

Commonly, AAVs are referred to in terms of their serotype. A serotype corresponds to a variant subspecies of AAV which, owing to its profile of expression of capsid surface antigens, has a distinctive reactivity which can be used to distinguish it from other variant subspecies. Typically, a virus having a particular AAV serotype does not efficiently cross-react with neutralising antibodies specific for any other AAV serotype.

AAV serotypes include AAV1, AAV2, AAV3, AAV4, AAV5, AAV6, AAV7, AAV8, AAV9, AAV10 and AAV11, and also recombinant serotypes, such as Rec2 and Rec3, recently identified from primate brain. Any of these AAV serotypes may be used in the invention. Thus, in one embodiment of the invention, the AAV vector particle is an AAV1, AAV2, AAV3, AAV4, AAV5, AAV6, AAV7, AAV8, AAV9, AAV10, AAV11, Rec2 or Rec3 AAV vector particle.

In one embodiment the AAV may be an AAV1, AAV2, AAV5, AAV7, AAV8 or AAV8 serotype.

In one embodiment the AAV may be an AAV2 or AAV8 serotype.

The capsid protein may be a mutant capsid protein such as disclosed in WO 2008/124724.

In one embodiment, the AAV vector comprises an AAV8 capsid with an Y733F mutation.

Reviews of AAV serotypes may be found in Choi et al. (2005) Curr. Gene Ther. 5: 299-310 and Wu et al. (2006) Molecular Therapy 14: 316-27. The sequences of AAV genomes or of elements of AAV genomes including ITR sequences, rep or cap genes for use in the invention may be derived from the following accession numbers for AAV whole genome sequences: Adeno-associated virus 1 NC_002077, AF063497; Adeno-associated virus 2 NC_001401; Adeno-associated virus 3 NC_001729; Adeno-associated virus 3B NC_001863; Adeno-associated virus 4 NC_001829; Adeno-associated virus 5 Y18065, AF085716; Adeno-associated virus 6 NC_001862; Avian AAV ATCC VR-865 AY186198, AY629583, NC_004828; Avian AAV strain DA-1 NC_006263, AY629583; Bovine AAV NC_005889, AY388617.

AAV may also be referred to in terms of clades or clones. This refers to the phylogenetic relationship of naturally derived AAVs, and typically to a phylogenetic group of AAVs which can be traced back to a common ancestor, and includes all descendants thereof. Additionally, AAVs may be referred to in terms of a specific isolate, i.e. a genetic isolate of a specific AAV found in nature. The term genetic isolate describes a population of AAVs which has undergone limited genetic mixing with other naturally occurring AAVs, thereby defining a recognisably distinct population at a genetic level.

The skilled person can select an appropriate serotype, clade, clone or isolate of AAV for use in the invention on the basis of their common general knowledge. For instance, the AAV5 capsid has been shown to transduce primate cone photoreceptors efficiently as evidenced by the successful correction of an inherited colour vision defect (Mancuso et al. (2009) Nature 461: 784-7).

The AAV serotype determines the tissue specificity of infection (or tropism) of an AAV virus. Accordingly, preferred AAV serotypes for use in AAVs administered to patients in accordance with the invention are those which have natural tropism for or a high efficiency of infection of target cells within the eye. In one embodiment, AAV serotypes for use in the invention are those which transduce cells of the neurosensory retina, retinal pigment epithelium and/or choroid.

Typically, the AAV genome of a naturally derived serotype, isolate or clade of AAV comprises at least one inverted terminal repeat sequence (ITR). An ITR sequence acts in cis to provide a functional origin of replication and allows for integration and excision of the vector from the genome of a cell. In preferred embodiments, one or more ITR sequences flank the nucleotide sequences encoding the Factor I (or fragments). The AAV genome typically also comprises packaging genes, such as rep and/or cap genes which encode packaging functions for an AAV particle. The rep gene encodes one or more of the proteins Rep78, Rep68, Rep52 and Rep40 or variants thereof. The cap gene encodes one or more capsid proteins such as VP1, VP2 and VP3 or variants thereof. These proteins make up the capsid of an AAV particle. Capsid variants are discussed below.

A promoter will be operably linked to each of the packaging genes. Specific examples of such promoters include the p5, p19 and p40 promoters (Laughlin et al. (1979) Proc. Natl. Acad. Sci. USA 76: 5567-5571). For example, the p5 and p19 promoters are generally used to express the rep gene, while the p40 promoter is generally used to express the cap gene.

As discussed above, the AAV genome used in the AAV vector of the invention may therefore be the full genome of a naturally occurring AAV. For example, a vector comprising a full AAV genome may be used to prepare an AAV vector or vector particle in vitro. However, while such a vector may in principle be administered to patients, this will rarely be done in practice. Preferably the AAV genome will be derivatised for the purpose of administration to patients. Such derivatisation is standard in the art and the invention encompasses the use of any known derivative of an AAV genome, and derivatives which could be generated by applying techniques known in the art. Derivatisation of the AAV genome and of the AAV capsid are reviewed in Coura and Nardi (2007) Virology Journal 4: 99, and in Choi et al. and Wu et al., referenced above.

Derivatives of an AAV genome include any truncated or modified forms of an AAV genome which allow for expression of a transgene from an AAV vector of the invention in vivo.

Typically, it is possible to truncate the AAV genome significantly to include minimal viral sequence yet retain the above function. This is preferred for safety reasons to reduce the risk of recombination of the vector with wild-type virus, and also to avoid triggering a cellular immune response by the presence of viral gene proteins in the target cell.

Typically, a derivative will include at least one inverted terminal repeat sequence (ITR), preferably more than one ITR, such as two ITRs or more. One or more of the ITRs may be derived from AAV genomes having different serotypes, or may be a chimeric or mutant ITR. A preferred mutant ITR is one having a deletion of a trs (terminal resolution site). This deletion allows for continued replication of the genome to generate a single-stranded genome which contains both coding and complementary sequences, i.e. a self-complementary AAV genome. This allows for bypass of DNA replication in the target cell, and so enables accelerated transgene expression.

The one or more ITRs will preferably flank the nucleotide sequence encoding the Factor I (or fragment thereof) at either end. The inclusion of one or more ITRs is preferred to aid concatamer formation of the vector of the invention in the nucleus of a host cell, for example following the conversion of single-stranded vector DNA into double-stranded DNA by the action of host cell DNA polymerases. The formation of such episomal concatamers protects the vector construct during the life of the host cell, thereby allowing for prolonged expression of the transgene in vivo.

In preferred embodiments, ITR elements will be the only sequences retained from the native AAV genome in the derivative. Thus, a derivative will preferably not include the rep and/or cap genes of the native genome and any other sequences of the native genome. This is preferred for the reasons described above, and also to reduce the possibility of integration of the vector into the host cell genome. Additionally, reducing the size of the AAV genome allows for increased flexibility in incorporating other sequence elements (such as regulatory elements) within the vector in addition to the transgene.

The following portions could therefore be removed in a derivative of the invention: one inverted terminal repeat (ITR) sequence, the replication (rep) and capsid (cap) genes. However, in some embodiments, derivatives may additionally include one or more rep and/or cap genes or other viral sequences of an AAV genome. Naturally occurring AAV integrates with a high frequency at a specific site on human chromosome 19, and shows a negligible frequency of random integration, such that retention of an integrative capacity in the vector may be tolerated in a therapeutic setting.

Where a derivative comprises capsid proteins i.e. VP1, VP2 and/or VP3, the derivative may be a chimeric, shuffled or capsid-modified derivative of one or more naturally occurring AAVs. In particular, the invention encompasses the provision of capsid protein sequences from different serotypes, clades, clones, or isolates of AAV within the same vector (i.e. a pseudotyped vector).

Chimeric, shuffled or capsid-modified derivatives will be typically selected to provide one or more desired functionalities for the AAV vector. Thus, these derivatives may display increased efficiency of gene delivery, decreased immunogenicity (humoral or cellular), an altered tropism range and/or improved targeting of a particular cell type compared to an AAV vector comprising a naturally occurring AAV genome, such as that of AAV2. Increased efficiency of gene delivery may be effected by improved receptor or co-receptor binding at the cell surface, improved internalisation, improved trafficking within the cell and into the nucleus, improved uncoating of the viral particle and improved conversion of a single-stranded genome to double-stranded form. Increased efficiency may also relate to an altered tropism range or targeting of a specific cell population, such that the vector dose is not diluted by administration to tissues where it is not needed.

Chimeric capsid proteins include those generated by recombination between two or more capsid coding sequences of naturally occurring AAV serotypes. This may be performed for example by a marker rescue approach in which non-infectious capsid sequences of one serotype are co-transfected with capsid sequences of a different serotype, and directed selection is used to select for capsid sequences having desired properties. The capsid sequences of the different serotypes can be altered by homologous recombination within the cell to produce novel chimeric capsid proteins.

Chimeric capsid proteins also include those generated by engineering of capsid protein sequences to transfer specific capsid protein domains, surface loops or specific amino acid residues between two or more capsid proteins, for example between two or more capsid proteins of different serotypes.

Shuffled or chimeric capsid proteins may also be generated by DNA shuffling or by error-prone PCR. Hybrid AAV capsid genes can be created by randomly fragmenting the sequences of related AAV genes e.g. those encoding capsid proteins of multiple different serotypes and then subsequently reassembling the fragments in a self-priming polymerase reaction, which may also cause crossovers in regions of sequence homology. A library of hybrid AAV genes created in this way by shuffling the capsid genes of several serotypes can be screened to identify viral clones having a desired functionality. Similarly, error prone PCR may be used to randomly mutate AAV capsid genes to create a diverse library of variants which may then be selected for a desired property.

The sequences of the capsid genes may also be genetically modified to introduce specific deletions, substitutions or insertions with respect to the native wild-type sequence. In particular, capsid genes may be modified by the insertion of a sequence of an unrelated protein or peptide within an open reading frame of a capsid coding sequence, or at the N- and/or C-terminus of a capsid coding sequence.

The unrelated protein or peptide may advantageously be one which acts as a ligand for a particular cell type, thereby conferring improved binding to a target cell or improving the specificity of targeting of the vector to a particular cell population. An example might include the use of RGD peptide to block uptake in the retinal pigment epithelium and thereby enhance transduction of surrounding retinal tissues (Cronin et al. (2008) ARVO Abstract: D1048). The unrelated protein may also be one which assists purification of the viral particle as part of the production process, i.e. an epitope or affinity tag. The site of insertion will typically be selected so as not to interfere with other functions of the viral particle e.g. internalisation, trafficking of the viral particle. The skilled person can identify suitable sites for insertion based on their common general knowledge. Particular sites are disclosed in Choi et al., referenced above.

The invention additionally encompasses the provision of sequences of an AAV genome in a different order and configuration to that of a native AAV genome. The invention also encompasses the replacement of one or more AAV sequences or genes with sequences from another virus or with chimeric genes composed of sequences from more than one virus. Such chimeric genes may be composed of sequences from two or more related viral proteins of different viral species.

The AAV vector of the invention may take the form of a nucleotide sequence comprising an AAV genome or derivative thereof and a sequence encoding the Factor I transgene.

The AAV particles of the invention include transcapsidated forms wherein an AAV genome or derivative having an ITR of one serotype is packaged in the capsid of a different serotype. The AAV particles of the invention also include mosaic forms wherein a mixture of unmodified capsid proteins from two or more different serotypes makes up the viral capsid. The AAV particle also includes chemically modified forms bearing ligands adsorbed to the capsid surface. For example, such ligands may include antibodies for targeting a particular cell surface receptor.

Thus, for example, the AAV particles of the invention include those with an AAV2 genome and AAV2 capsid proteins (AAV2/2), those with an AAV2 genome and AAV5 capsid proteins (AAV2/5) and those with an AAV2 genome and AAV8 capsid proteins (AAV2/8), as well as those with an AAV2 genome and capsid proteins of more than one serotype.

The AAV vector may comprise multiple copies (e.g., 2, 3 etc) of the nucleotide sequence referred to herein.

### Promoters and regulatory sequences

The AAV vector of the invention may also include elements allowing for the expression of the Factor I transgene (or fragment thereof) in vitro or in vivo. These may be referred to as expression control sequences. Thus, the AAV vector typically comprises expression control sequences (e.g. comprising a promoter sequence) operably linked to the nucleotide sequence encoding the transgene.

Any suitable promoter may be used, the selection of which may be readily made by the skilled person. The promoter sequence may be constitutively active (i.e. operational in any host cell background), or alternatively may be active only in a specific host cell environment, thus allowing for targeted expression of the transgene in a particular cell type (e.g. a tissue-specific promoter). The promoter may show inducible expression in response to presence of another factor, for example a factor present in a host cell. In any event, where the vector is administered for therapy, it is preferred that the promoter should be functional in the target cell background.

In some embodiments, it is preferred that the promoter shows retinal-cell specific expression in order to allow for the transgene to only be expressed in retinal cell populations. Thus, expression from the promoter may be retinal-cell specific, for example confined only to cells of the neurosensory retina and retinal pigment epithelium.

Preferred promoters, which are not retinal-cell specific, include the chicken beta-actin (CBA) promoter, optionally in combination with a cytomegalovirus (CMV) enhancer element. An example promoter for use in the invention is a CAG promoter, for example the promoter used in the rAVE expression cassette (GeneDetect.com). A further example promoter for use in the invention has the sequence:

ATTGACGTCAATAATGACGTATGTTCCCATAGTAACGCCAATAGGGACTTTCCATTGACGTCAATGGGTG
GAGTATTTACGGTAAACTGCCCACTTGGCAGTACATCAAGTGTATCATATGCCAAGTACGCCCCCTATTG
ACGTCAATGACGGTAAATGGCCCGCCTGGCATTATGCCCAGTACATGACCTTATGGGACTTTCCTACTTG
GCAGTACATCTACGTATTAGTCATCGCTATTACCATGGTCGAGGTGAGCCCCACGTTCTGCTTCACTCTC
CCCATCTCCCCCCCCTCCCCACCCCCAATTTTGTATTTATTTATTTTTTAATTATTTTGTGCAGCGATGG
GGGCGGGGGGGGGGGGGGGGCGCGCGCCAGGCGGGGCGGGGCGGGGCGAGGGGCGGGGCGGGGCGAGGCG

GAGAGGTGCGGCGGCAGCCAATCAGAGCGGCGCGCTCCGAAAGTTTCCTTTTATGGCGAGGCGGCGGCGG
CGGCGGCCCTATAAAAAGCGAAGCGCGCGGCGGGCGGGAGTCGCTGCGCGCTGCCTTCGCCCCGTGCCCC
GCTCCGCCGCCGCCTCGCGCCGCCCGCCCCGGCTCTGACTGACCGCGTTACTCCCACAGGTGAGCGGGCG
GGACGGCCCTTCTCCTCCGGGCTGTAATTAGCGCTTGGTTTAATGACGGCTTGTTTCTTTTCTGTGGCTG
CGTGAAAGCCTTGAGGGGCTCCGGGAGGGCCCTTTGTGCGGGGGGAGCGGCTCGGGGCTGTCCGCGGGGG
GACGGCTGCCTTCGGGGGGGACGGGGCAGGGCGGGGTTCGGCTTCTGGCGTGTGACCGGCGGCTCTAGAG
CCTCTGCTAACCATGTTCATGCCTTCTTCTTTTTCCTACAGCTCCTGGGCAACGTGCTGGTTATTGTGCT
GTCTCATCATTTTGGCAAAGAATT

(SEQ ID NO: 5)

In one embodiment, the AAV vector comprises a promoter with the nucleotide sequence of SEQ ID NO: 5. In another embodiment, the AAV vector comprises a promoter with a nucleotide sequence that has at least 70%, 80%, 90%, 95%, 96%, 97%, 98% 99% or 100% identity to SEQ ID NO: 5, wherein the nucleotide sequence substantially retains the functional activity of the promoter represented by SEQ ID NO: 5.

Examples of promoters based on human sequences that would induce retina-specific gene expression include rhodopsin kinase for rods and cones (Allocca et al. (2007) J. Virol. 81: 11372-80), PR2.1 for cones only (Mancuso et al. (2009) Nature 461: 784-7) and/or RPE65 (Bainbridge et al. (2008) N. Engl. J. Med. 358: 2231-9) or VMD2 (Esumi et al. (2004) J. Biol. Chem. 279: 19064-73) for the retinal pigment epithelium.

The AAV vector of the invention may also comprise one or more additional regulatory sequences which may act pre- or post-transcriptionally. The regulatory sequence may be part of the native transgene locus or may be a heterologous regulatory sequence. The AAV vector of the invention may comprise portions of the 5'-UTR or 3'-UTR from the native transgene transcript.

Regulatory sequences are any sequences which facilitate expression of the transgene, i.e. act to increase expression of a transcript, improve nuclear export of mRNA or enhance its stability. Such regulatory sequences include for example enhancer elements, post-transcriptional regulatory elements and polyadenylation sites. A preferred polyadenylation site is the Bovine Growth Hormone poly-A signal which may be as shown below:

TCGCTGATCAGCCTCGACTGTGCCTTCTAGTTGCCAGCCATCTGTTGTTTGCCCCTCCCCCGTGCCTTCC
TTGACCCTGGAAGGTGCCACTCCCACTGTCCTTTCCTAATAAAATGAGGAAATTGCATCGCATTGTCTGA
GTAGGTGTCATTCTATTCTGGGGGGTGGGGTGGGGCAGGACAGCAAGGGGGAGGATTGGGAAGACAATAG
CAGGCATGCTGGGGATGCGGTGGGCTCTATGGCTTCTGAGGCGGAAAGAACCAGCTGGGG

(SEQ ID NO: 6)

In one embodiment, the AAV vector comprises a polyadenylation site with the nucleotide sequence of SEQ ID NO: 6. In another embodiment, the AAV vector comprises a polyadenylation site with a nucleotide sequence that has at least 70%, 80%, 90%, 95%, 96%, 97%, 98% 99% or 100% identity to SEQ ID NO: 6, wherein the nucleotide sequence substantially retains the functional activity of the polyadenylation site represented by SEQ ID NO: 6.

In the context of the AAV vector of the invention, such regulatory sequences will be cis-acting. However, the invention also encompasses the use of trans-acting regulatory sequences located on additional genetic constructs.

A preferred post-transcriptional regulatory element for use in a AAV vector of the invention is the woodchuck hepatitis post-transcriptional regulatory element (WPRE) or a variant thereof. An example sequence of the WPRE is shown below:

ATCAACCTCTGGATTACAAAATTTGTGAAAGATTGACTGGTATTCTTAACTATGTTGCTCCTTTTACGCT
ATGTGGATACGCTGCTTTAATGCCTTTGTATCATGCTATTGCTTCCCGTATGGCTTTCATTTTCTCCTCC
TTGTATAAATCCTGGTTGCTGTCTCTTTATGAGGAGTTGTGGCCCGTTGTCAGGCAACGTGGCGTGGTGT
GCACTGTGTTTGCTGACGCAACCCCCACTGGTTGGGGCATTGCCACCACCTGTCAGCTCCTTTCCGGGAC
TTTCGCTTTCCCCCTCCCTATTGCCACGGCGGAACTCATCGCCGCCTGCCTTGCCCGCTGCTGGACAGGG
GCTCGGCTGTTGGGCACTGACAATTCCGTGGTGTTGTCGGGGAAATCATCGTCCTTTCCTTGGCTGCTCG
CCTGTGTTGCCACCTGGATTCTGCGCGGGACGTCCTTCTGCTACGTCCCTTCGGCCCTCAATCCAGCGGA
CCTTCCTTCCCGCGGCCTGCTGCCGGCTCTGCGGCCTCTTCCGCGTCTTCGCCTTCGCCCTCAGACGAGT
CGGATCTCCCTTTGGGCCGCCTCCCCGC

(SEQ ID NO: 7)

In one embodiment, the AAV vector comprises a post-transcriptional regulatory element with the nucleotide sequence of SEQ ID NO: 7. In another embodiment, the AAV vector comprises a post-transcriptional regulatory element with a nucleotide sequence that has at least 70%, 80%, 90%, 95%, 96%, 97%, 98% 99% or 100% identity to SEQ ID NO: 7, wherein the nucleotide sequence substantially retains the functional activity of the post-transcriptional regulatory element represented by SEQ ID NO: 7.

The invention encompasses the use of any variant sequence of the WPRE which increases expression of the transgene compared to a AAV vector without a WPRE. Preferably, variant sequences display at least 70% identity to SEQ ID NO: 7 over its entire sequence, more preferably 75%, 80%, 85%, 90% and more preferably at least 95%, 96% 97%, 98% or 99% identity to SEQ ID NO: 7 over its entire sequence.

Another regulatory sequence which may be used in a AAV vector of the invention is a scaffold-attachment region (SAR). Additional regulatory sequences may be readily selected by the skilled person.

### Method of administration

In a preferred embodiment, the AAV vector is administered intraocularly.

The term "intraocular" refers to the interior of the eye, thus intraocular administration relates to the administration to the interior of the eye of a subject

In one embodiment the AAV vector is administered to the eye of a subject by subretinal, direct retinal, suprachoroidal or intravitreal injection. In one embodiment said administration is performed by a robot.

The volume of the medicament composition injected may, for example, be about 10-500 µL, for example about 50-500, 100-500, 200-500, 300-500, 400-500, 50-250, 100-250, 200-250 or 50-150 µL. The volume may, for example, be about 10, 50, 100, 150, 200, 250, 300, 350, 400, 450 or 500 µL. Preferably, the volume of the medicament composition injected is 100 µL.

The skilled person will be familiar with and well able to carry out individual subretinal, direct retinal, suprachoroidal or intravitreal injections.

Preferably, the AAV vector is administered by subretinal injection.

In one embodiment, the AAV vector or pharmaceutical composition comprising the same is administered not more than once, or not more than twice, during the lifetime of a subject.

### Subretinal injection

Subretinal injections are injections into the subretinal space, i.e. underneath the neurosensory retina. During a subretinal injection, the injected material is directed into, and creates a space between, the photoreceptor cell and retinal pigment epithelial (RPE) layers. When the injection is carried out through a small retinotomy, a retinal detachment may be created. The detached, raised layer of the retina that is generated by the injected material is referred to as a "bleb".

The hole created by the subretinal injection must be sufficiently small that the injected solution does not significantly reflux back into the vitreous cavity after administration. Such reflux would be particularly problematic when a medicament is injected, because the effects of the medicament would be directed away from the target zone. Preferably, the injection creates a self-sealing entry point in the neurosensory retina, i.e. once the injection needle is removed, the hole created by the needle reseals such that very little or substantially no injected material is released through the hole.

To facilitate this process, specialist subretinal injection needles are commercially available (e.g. DORC 41G Teflon subretinal injection needle, Dutch Ophthalmic Research Center International BV, Zuidland, The Netherlands). These are needles designed to carry out subretinal injections.

Unless damage to the retina occurs during the injection, and as long as a sufficiently small needle is used, substantially all injected material remains localised between the detached neurosensory retina and the RPE at the site of the localised retinal detachment (i.e. does not reflux into the vitreous cavity). Indeed, the typical persistence of the bleb over a short time frame indicates that there is usually little escape of the injected material into the vitreous. The bleb may dissipate over a longer time frame as the injected material is absorbed.

Visualisations of the eye, in particular the retina, for example using optical coherence tomography, may be made pre-operatively.

The volume of the medicament composition injected may, for example, be about 10-500 µL, for example about 50-500, 100-500, 200-500, 300-500, 400-500, 50-250, 100-250, 200-250 or 50-150 µL. The volume may, for example, be about 10, 50, 100, 150, 200, 250, 300, 350, 400, 450 or 500 µL. Preferably, the volume of the medicament composition injected is 100 µL. Larger volumes may increase the risk of stretching the retina, while smaller volumes may be difficult to see.

### Two-step subretinal injection

The vector of the invention may be delivered with increased accuracy and safety by using a two-step method in which a localised retinal detachment is created by the subretinal injection of a first solution. The first solution does not comprise the vector. A second subretinal injection is then used to deliver the medicament comprising the vector into the subretinal fluid of the bleb created by the first subretinal injection. Because the injection delivering the medicament is not being used to detach the retina, a specific volume of solution may be injected in this second step.

In one embodiment the subretinal injection of the vector comprises the steps:
(a) administering a solution to the subject by subretinal injection in an amount effective to at least partially detach the retina to form a subretinal bleb, wherein the solution does not comprise the vector; and
(b) administering a medicament composition by subretinal injection into the bleb formed by step (a), wherein the medicament comprises the vector.

The volume of solution injected in step (a) to at least partially detach the retina may be, for example, about 10-1000 µL, for example about 50-1000, 100-1000, 250-1000, 500-1000, 10-500, 50-500, 100-500, 250-500 µL. The volume may be, for example, about 10, 50, 100, 200, 300, 400, 500, 600, 700, 800, 900 or 1000 µL.

The volume of the medicament composition injected in step (b) may be, for example, about 10-500 µL, for example about 50-500, 100-500, 200-500, 300-500, 400-500, 50-250, 100-250, 200-250 or 50-150 µL. The volume may be, for example, about 10, 50, 100, 150, 200, 250, 300, 350, 400, 450 or 500 µL. Preferably, the volume of the medicament composition injected in step (b) is 100 µL. Larger volumes may increase the risk of stretching the retina, while smaller volumes may be difficult to see.

The solution that does not comprise the medicament (i.e. the "solution" of step (a)) may be similarly formulated to the solution that does comprise the medicament, as described below. A preferred solution that does not comprise the medicament is balanced saline solution (BSS) or a similar buffer solution matched to the pH and osmolality of the subretinal space.

### Visualising the retina during surgery

Under certain circumstances, for example during end-stage retinal degenerations, identifying the retina is difficult because it is thin, transparent and difficult to see against the disrupted and heavily pigmented epithelium on which it sits. The use of a blue vital dye (e.g. Brilliant Peel^{®}, Geuder; MembraneBlue-Dual^{®}, Dorc) may facilitate the identification of the retinal hole made for the retinal detachment procedure (i.e. step (a) in the two-step subretinal injection method of the invention) so that the medicament can be administered through the same hole without the risk of reflux back into the vitreous cavity.

The use of the blue vital dye also identifies any regions of the retina where there is a thickened internal limiting membrane or epiretinal membrane, as injection through either of these structures would hinder clean access into the subretinal space. Furthermore, contraction of either of these structures in the immediate post-operative period could lead to stretching of the retinal entry hole, which could lead to reflux of the medicament into the vitreous cavity.

### Suprachoroidal injection

The vector of the invention may be delivered to the suprachoroidal space using an ab externo approach that utilises an microcatheter (see, for example, Peden et al. (2011) PLoS One 6(2): e17140). In this method a limbal conjunctival peritomy is performed to expose bare sclera, followed by sclerotomy to expose bare choroid. A microcatheter (such as the iTrack 250A from iScience Interventional, optionally connected to an illumination system such as the iLumin laser-diode based micro-illumination system (iScience Interventional)) is introduced into the suprachoroidal space and advanced posteriorly towards the optic disc. Following manipulation of the microcatheter tip into the desired position, injection of the vector forms a bleb within the retina and choroid.

Thus, in one embodiment, the vector is delivered suprachoroidally by a method comprising (i) introduction of a microcatheter into the suprachoroidal space; (ii) advancing the microcatheter within said space until the tip is in the proximity of the afflicted region of the retina; and (iii) injecting the vector from the microcatheter tip to create a bleb.

In one embodiment, the above administration procedures are directly carried out by a robot.

### Pharmaceutical compositions and injected solutions

The medicaments, for example AAV vectors, of the invention may be formulated into pharmaceutical compositions. These compositions may comprise, in addition to the medicament, a pharmaceutically acceptable carrier, diluent, excipient, buffer, stabiliser or other materials well known in the art. Such materials should be non-toxic and should not interfere with the efficacy of the active ingredient. The precise nature of the carrier or other material may be determined by the skilled person according to the route of administration, e.g. subretinal, direct retinal, suprachoroidal or intravitreal injection.

The pharmaceutical composition is typically in liquid form. Liquid pharmaceutical compositions generally include a liquid carrier such as water, petroleum, animal or vegetable oils, mineral oil or synthetic oil. Physiological saline solution, magnesium chloride, dextrose or other saccharide solution, or glycols such as ethylene glycol, propylene glycol or polyethylene glycol may be included. In some cases, a surfactant, such as pluronic acid (PF68) 0.001 % may be used.

For injection at the site of affliction, the active ingredient may be in the form of an aqueous solution which is pyrogen-free, and has suitable pH, isotonicity and stability. The skilled person is well able to prepare suitable solutions using, for example, isotonic vehicles such as Sodium Chloride Injection, Ringer's Injection or Lactated Ringer's Injection. Preservatives, stabilisers, buffers, antioxidants and/or other additives may be included as required.

For delayed release, the medicament may be included in a pharmaceutical composition which is formulated for slow release, such as in microcapsules formed from biocompatible polymers or in liposomal carrier systems according to methods known in the art.

### Method of treatment

It is to be appreciated that all references herein to treatment include curative, palliative and prophylactic treatment; although in the context of the invention references to preventing are more commonly associated with prophylactic treatment. Treatment may also include arresting progression in the severity of a disease.

The treatment of mammals, particularly humans, is preferred. However, both human and veterinary treatments are within the scope of the disclosure.

Any references to methods for treatment of the human or animal body by surgery or therapy are to be interpreted as references to the compounds, pharmaceutical compositions and medicaments of the present invention for use in a method for treatment.

### Variants, derivatives, analogues, homologues and fragments

In addition to the specific proteins and nucleotides mentioned herein, also described herein is the use of variants, derivatives, analogues, homologues and fragments thereof.

In the context of the disclosure, a variant of any given sequence is a sequence in which the specific sequence of residues (whether amino acid or nucleic acid residues) has been modified in such a manner that the polypeptide or polynucleotide in question substantially retains its function. A variant sequence can be obtained by addition, deletion, substitution, modification, replacement and/or variation of at least one residue present in the naturally-occurring protein.

The term "derivative" as used herein, in relation to proteins or polypeptides described herein includes any substitution of, variation of, modification of, replacement of, deletion of and/or addition of one (or more) amino acid residues from or to the sequence providing that the resultant protein or polypeptide substantially retains at least one of its endogenous functions. The term "analogue" as used herein, in relation to polypeptides or polynucleotides includes any mimetic, that is, a chemical compound that possesses at least one of the endogenous functions of the polypeptides or polynucleotides which it mimics.

Typically, amino acid substitutions may be made, for example from 1, 2 or 3 to 10 or 20 substitutions provided that the modified sequence substantially retains the required activity or ability. Amino acid substitutions may include the use of non-naturally occurring analogues.

Proteins used in the disclosure may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I LV |
| | Polar - uncharged | C S T M |
| | | N Q |
| | Polar - charged | D E |
| | | K R H |
| AROMATIC | | F W Y |

The term "homologue" as used herein means an entity having a certain homology with the wild type amino acid sequence and the wild type nucleotide sequence. The term "homology" can be equated with "identity".

A homologous sequence may include an amino acid sequence which may be at least 50%, 55%, 60%, 65%, 70%, 75%, 80%, 85% or 90% identical, preferably at least 95% or 97% or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the invention it is preferred to express homology in terms of sequence identity.

A homologous sequence may include a nucleotide sequence which may be at least 50%, 55%, 60%, 65%, 70%, 75%, 80%, 85% or 90% identical, preferably at least 95% or 97% or 99% identical to the subject sequence. Although homology can also be considered in terms of similarity, in the context of the invention it is preferred to express homology in terms of sequence identity.

Preferably, reference to a sequence which has a percent identity to any one of the SEQ ID NOs detailed herein refers to a sequence which has the stated percent identity over the entire length of the SEQ ID NO referred to.

Homology comparisons can be conducted by eye or, more usually, with the aid of readily available sequence comparison programs. These commercially available computer programs can calculate percentage homology or identity between two or more sequences.

Percentage homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the nucleotide sequence may cause the following codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum percentage homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al. (1984) Nucleic Acids Res. 12: 387). Examples of other software that can perform sequence comparisons include, but are not limited to, the BLAST package (see Ausubel et al. (1999) ibid - Ch. 18), FASTA (Atschul et al. (1990) J. Mol. Biol. 403-410) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, called BLAST 2 Sequences is also available for comparing protein and nucleotide sequences (see FEMS Microbiol. Lett. (1999) 174: 247-50; FEMS Microbiol. Lett. (1999) 177: 187-8).

Although the final percent homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62.

Once the software has produced an optimal alignment, it is possible to calculate percent homology, preferably percent sequence identity. The software typically does this as part of the sequence comparison and generates a numerical result.

"Fragments" of full length Factor I or Factor H are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

Various preferred features and embodiments of the present invention will now be described by way of non-limiting examples.

The practice of the present invention will employ, unless otherwise indicated, conventional techniques of chemistry, biochemistry, molecular biology, microbiology and immunology, which are within the capabilities of a person of ordinary skill in the art. Such techniques are explained in the literature. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press.

### EXAMPLES

### Example 1

### Cloning of human CFI cDNA, and generation of the CBA-CFI-WPRE expression cassette, construction of pAAV-CBA-CFI-WPRE-bGHpA and packaging of AAV-CFI virus.

The cDNA of the most common human CFI sequence variant was downloaded from Genbank, Accession Number NM_000204.4. The cDNA has the sequence of SEQ ID NO: 2 and was ordered as gBlocks^{®} Gene Fragments from Integrated DNA Technologies. A second CFI construct was also ordered in which the cDNA sequence of the CFI gene was codon-optimised for expression in human cells. The codon-optimised sequence ("CFlco") has the sequence of SEQ ID NO: 8 and was ordered from GeneWiz (CFlco in plasmid pUC57).

These cDNA sequences were inserted into a pAAV cis plasmid, termed pAM. pAM is a high copy number plasmid originally derived from pBR322, but includes stabilized AAV-2 left and right inverted terminal repeats which flank the expression cassette of choice. For the AAV-CFI and AAV-CFlco vector, a modified CBA/CAG promoter (chicken beta-actin with CMV enhancer; called "CBA" herein) was used to drive expression of CFI and CFlco and a modified WPRE sequence and bGH polyA were provided 3' to the cDNA. The two plasmids were termed pAAV2.CBA-hCFI-WPRE-bGH, (pAAV-CFI), and pAAV2.CBA-hCFIco-WPRE-bGH, (pAAV-CFlco).

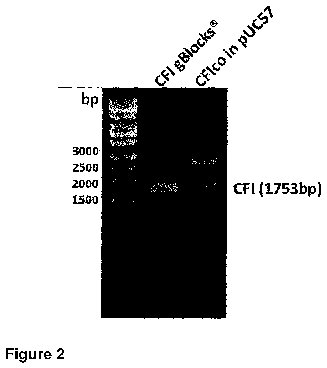

Figure 2 shows an agarose gel of restriction digests of CFI and CFlco. The 1752bp band of CFI was excised and cloned into the pAAV-CBA- WPRE-bGHpA backbone.

### Comparison of CFI expression levels of pAAV.CFI with pAAV.CFlco, co-transfection of ARPE-19 cell line with pAAV-CFI or pAAV.CFlco and pCMV.GFP, and immunoblotting of CFI.

ARPE-19 (ATCC^{®} CRL-2302^{™}) is a spontaneously arising retinal pigment epithelia (RPE) cell line derived in 1986 by Amy Aotaki-Keen from the normal eyes of a 19-year-old male who died from head trauma in a motor vehicle accident. These cells form stable adherent monolayers, which exhibit morphological polarization when plated on laminin-coated Transwell-COL filters in medium with a low serum concentration. ARPE-19 cells were acquired from the American Type Culture Collection (ATCC). Cells were grown in DMEM/F12 (Thermo Fisher Scientific) supplemented with 10% heat-inactivated fetal bovine serum (Gibco), 1% 200mM L-Glutamine (Sigma Aldrich) and 1% Penicillin-Streptomycin (Sigma Aldrich, 10,000 units penicillin, 10mg streptomycin/ml).

Co-transfection of ARPE-19 was performed using Lipofectamine LTX (Life Technologies) according to manufacturer's protocol. 0.5µg of pAAV-CFI or pAAV.CFlco were co-transfected with 0.5µg of pCMV.GFP (Plasmid Factory). Co-transfection of a pAAV-CBA-WPRE-bGHpA (pAAV [without transgene cassette]) served as a negative control. 24 hours post transfection, cells were washed with PBS (Phosphate Buffered Saline, pH 7.2, Gibco^{™}) and cultured in serum-free growth medium for 72 hours. Supernatant was taken, cleared by centrifugation and stored at -80 °C. ARPE-19 cells were detached with TrypLE Express (Gibco) and counted to ensure equal numbers of cells per well. The pellet was frozen at -20 °C for 30 minutes. 1xRIPA lysis buffer (Merck Millipore) supplemented with cOmplete^{™} EDTA-free Protease Inhibitor Cocktail (Roche) was added to the pellet and cells were disrupted by sonication. Insoluble protein was centrifuged down and the supernatant (= lysate) was stored at -80 °C.

Immunoblotting of CFI was performed by loading 30µl of undiluted supernatant in 4x Laemmli buffer (250mM Tris-HCl (pH 6.8), 8% SDS, 40% glycerol, and 0.02% bromophenol blue) and separation of proteins on a 10% precast polyacrylamide gel (Bio-Rad). Proteins were blotted to a PVDF membrane (Bio-Rad) by semi-dry transfer after which the membrane was blocked in blocking buffer (1xTBS pH 8 [Sigma], 0.05% Tween-20 and 5% dried skimmed milk powder [Marvel]). CFI was detected with a OX21 (Table 1) and an anti-mouse IgG HRP conjugated antibody (Table 1) diluted in blocking buffer. Protein bands were visualised using Clarity Western ECL Substrate (Bio-Rad) and analysed with an Odyssey^{®} Fc Imaging System. Immunoblotting of GFP was performed by loading 5µl of undiluted supernatant in 4x Laemmli buffer supplemented with 50µl/ml β-Mercaptoethanol. Immunoblot was performed as described above. GFP was detected with an antibody to TurboGFP (Table 1) and an anti-rabbit IgG HRP conjugated antibody (Table 1).

**Table 1. Primary and secondary antibodies used for immunoblotting or immunoprecipitation.**

| **Name/target** | **Class and host** | **Provider** | **Dilution** |
|---|---|---|---|
| Ox21/human CFI | Monoclonal, mouse | Thermo Fisher Scientific | 1:500-1000 |
| Turbo-GFP | Polyclonal, rabbit | Thermo Fisher Scientific | 1:5000 |
| Anti-Human Factor I | Antiserum, goat | Comptech | 1:1000-5000 |
| Anti-β-actin | Monoclonal, mouse | Abcam | 1:20,000 |
| Clone 9/human C3g* | Monoclonal, rat | Hycult | 0.5µg/ml |
| Anti-mouse IgG HRP conjugated | Polyclonal, donkey | Abcam | 1:2500-5000 |
| Anti-rabbit IgG HRP conjugated | Polyclonal, donkey | Abcam | 1:5000 |
| Anti-goat IgG HRP conjugated | Polyclonal, rabbit | Sigma | 1:5000 |
| Extravidin-HRP | | Sigma | 1:5000 I |

| | | | |
|---|---|---|---|
| * Clone 9 antibody was biotinylated using EZ-Link^{™} NHS-LC-LC-Biotin (Thermo Fisher Scientific) | | | |

Figure 3 shows immunoblotting of CFI (Figure 3A) and of GFP (Figure 3B). 3A: CFI appears as a 70kDa band (non-reduced) and was expressed at equal rates after transfection of ARPE-19 with pAAV.CFI or pAAV.CFlco. No CFI was expressed after transfection with pAAV. 10% normal human serum (NHS) was used as a positive control for CFI immunoblotting. 3B: Transfection efficiency was analysed by co-transfection of ARPE-19 cells with pCMV.GFP. GFP appears as a 30kDa band and the immunoblot confirmed that cells have been transfected at similar efficiencies.

### Preparation of AAV.CFI and AAV.CFlco.

AAV2 virus was prepared by double transfection of HEK-293 (ATCC^{®} CRL-1573^{™}) or HEK-293T (ATCC^{®} CRL-3216^{™}) cell lines (both adherent) with pAAV.CFI or pAAV.CFlco and pDG (Plasmid Factory) providing adenoviral helper sequences and packaging sequences (Rep/Cap). HEK-293 and HEK-293T cells were grown in DMEM (Sigma) supplemented with 10% heat-inactivated fetal bovine serum (Gibco), 1% 200mM L-Glutamine (Sigma Aldrich) and 1% Penicillin-Streptomycin (Sigma Aldrich, 10,000 units penicillin, 10mg streptomycin/ml). Cells were harvested after 72 hours and lysed to purify the virus particles. AAV particles were purified on an iodixanol gradient and recovered from the 40% fraction. Virus was concentrated on Amicon Ultra-15 centrifugal filter units (Merck Millipore) and stored in aliquots at -80°C. Virus purity was assessed by SDS PAGE and the titer was determined by qPCR.

**In vitro expression analysis of AAV.CFI, AAV.CFlco and AAV.GFP, transduction of HEK-293 and ARPE-19 cell lines, immunoblot of tissue culture supernatant to analyse CFI expression.**

70% confluent HEK-293 ("293") and ARPE-19 cell lines were transduced with AAV.CFI, AAV.CFlco and AAV.GFP with a multiplicity of infection (MOI) of 1x10⁴ in normal growth medium supplemented with only 1% heat-inactivated fetal bovine serum. After 7 days, supernatant was harvested and cleared by centrifugation. Supernatant was stored in aliquots at -80°C.

Immunoblotting was performed as described above and 30µl supernatant was mixed with 4x Laemmli buffer and loaded onto a 10% precast polyacrylamide gel. CFI was detected with OX21 (Table 1) and anti-mouse IgG HRP conjugated antibody (Table 1) diluted in blocking buffer (when supernatant was loaded under non-reducing conditions) or an antiserum to human CFI (Table 1) and anti-goat IgG (whole molecule) - peroxidase antibody (Table 1) (when supernatant was loaded under reducing conditions).

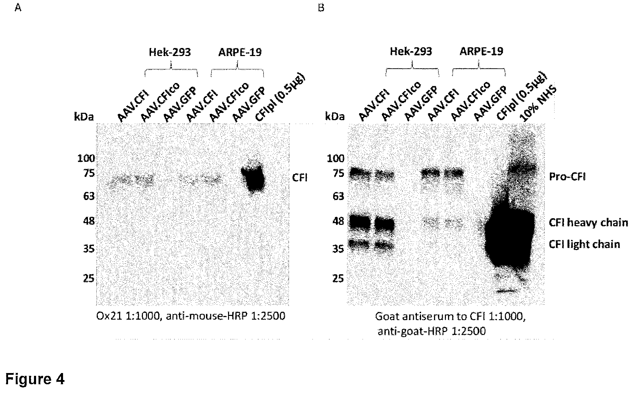

Figure 4 shows immunoblotting of CFI in supernatant of virus transduced HEK-293 and ARPE-19 cell lines. 4A: Supernatant was loaded under non-reducing conditions and CFI was detected with a mouse monoclonal antibody to human CFI (OX21, Thermo Fisher Scientific) and a donkey anti-mouse IgG HRP conjugated antibody (Abcam). CFI and CFlco were expressed in both cell lines. Transduction of cell lines with AAV.GFP served as a negative control while 0.5µg of plasma purified human CFI (called "CFlpl" herein) (Comptech) served as a positive control. 4B: Supernatant was loaded under reducing conditions and CFI was detected with a goat antiserum to human CFI (Comptech) and rabbit anti-goat IgG (whole molecule) - Peroxidase antibody (Sigma). CFI appeared at 80kDa (pro-enzyme), 50kDa (processed; heavy chain) and 35kDa (processed; light chain). AAV.GFP served as a negative control while 0.5µg of plasma purified human CFI (Comptech) and 10% normal human serum (called "NHS" herein) served as a positive control. CFI and CFlco were expressed in both cell lines.

### Qualitative analysis of CFI expression, C3b cleavage assay to measure functional activity

For qualitative analysis, CFI was immunoprecipitated using Pierce Co-Immunoprecipitation (Co-IP) Kit (Thermo Fisher Scientific) according to manufacturer's protocol. 30µg of affinity purified monoclonal antibody to human CFI (Ox21, Thermo Fisher Scientific) was incubated for 2 hours at room temperature with 25µl of AminoLink Plus Coupling Resin. Supernatant of HEK-293 or ARPE-19 transduced cells was incubated with the prepared resin overnight at 4°C. Next day, resin was washed several times using provided IP Lysis/Wash Buffer. Bound CFI was eluted with the kit's elution buffer (0.1M Glycine, pH 2.7) and immediately neutralised with 1M Tris, pH 9.5. Immunoprecipitation of CFI was evaluated by measuring the absorbance at 280nm and by SDS PAGE analysis. CFI was either used directly in a C3b cleavage assay or stored in aliquots at -80 °C.

In a C3b cleavage assay, 1mg of plasma purified C3b is incubated for 1 hour at 37 °C with 0.5µg of plasma purified Complement Factor H (CFH) and either plasma purified Complement Factor I (CFlpl) (all plasma purified proteins were acquired from Comptech), cell culture supernatant of AAV.CFI transduced cells or immunoprecipitated CFI/CFlco. 4x Laemmli buffer with β-mercapto-ethanol is added to stop the reaction. Samples were diluted and loaded on a 10% precast polyacrylamide SDS PAGE gel (Bio-Rad). After transfer to a PVDF membrane (Bio-Rad) and blocking in blocking buffer (1xTBS pH 8 [Sigma]/0.05% Tween-20 and 5% dried skimmed milk powder [Marvel]), C3b cleavage was detected with biotinylated clone 9 (Table 1) and Extravidin-HRP conjugated (Table 1). This antibody reacts with an epitope in C3g and on a reducing SDS PAGE recognises the α-chain of C3, the α' chain of C3b, the C3α'1-chain of iC3b and C3dg. It can therefore be used to analyse degradation of C3b by CFl. CFH serves as a co-factor for C3b degradation and because the assay is run at low-ionic strength, it also serves as a co-factor for the second cleavage of iC3b to C3dg. The buffer used was "elution buffer" of Pierce Co-Immunoprecipitation (Co-IP) Kit (Thermo Fisher Scientific) neutralised with 1M Tris, pH 9.5.

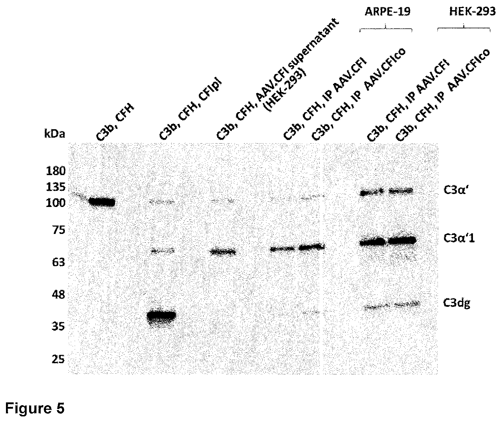

Figure 5 shows a representative result of a C3b cleavage assay. Lane 1 shows C3b incubated with CFH only. Lane 2 shows C3b incubated with CFH and CFlpl. C3b is degraded by CFlpl to iC3b and C3dg. Lane 3 shows C3b incubated with CFH and supernatant from HEK-293 transduced with AAV.CFI. Lane 4-5 show C3b incubated with CFH and immunoprecipitated CFI (designated as "IP CFI") from ARPE-19 cells transduced with either AAV.CFI (lane 4) or AAV.CFlco (lane 5). Lane 6-7 show C3b incubated with CFH and immunoprecipitated CFI from HEK-293 transduced with either AAV.CFI (lane 6) or AAV.CFlco (lane 7). CFI secreted from transduced cell lines is functionally active and cleaves the α-chain of C3b to iC3b. Immunoprecipitation of CFI leads to increase in total CFI amount which is reflected by the appearance of the C3dg band in addition to the α'1 fragment band of iC3b. This second cleavage only happens at low ionic strength and at a low rate, requiring more CFI to be present.

**In vitro analysis of CFI expression in transduced ARPE-19 grown on a permeable transwell filter.**

RPE cells are pigmented, non-dividing cells that exhibit morphological polarization when plated on laminin-coated Transwell-COL filters in medium with a low serum concentration. The Transwell filter (Polyester membrane inserts, pore diameter 0.4 µm, membrane diameter 12 mm) functions to separate the culture wells into two compartments, the apical (upper) domain corresponding to the retinal facing side of the RPE monolayer and basolateral (lower) domain corresponding to the choroidal facing side of the RPE monolayer. When left under low serum medium conditions, ARPE-19 cells will differentiate and become hexagonal, lightly pigmented cells. The transwell model can be used to assess expression in quiescent cells, to reflect situation in vivo.

Transwells were coated with 8µg/ml laminin (Sigma) and ARPE-19 cells were seeded in 10% FBS-medium as monolayers on transwells. After 48 hours medium was changed to 1% FBS medium and cells were transduced with an MOI 10⁵/cell (AAV.CFI, AAV.CFlco and AAV.GFP). After 7 days supernatant was harvested from both compartments and cleared by centrifugation. Because of the different volume in upper and lower compartment, i.e. 500µl medium in upper and 1500µl in lower compartment, supernatant was analysed normalised to total volume: 30µl supernatant from lower compartment were mixed with 4x Laemmli buffer and 10µl supernatant from upper compartment were mixed with 4x Laemmli buffer. Samples were loaded onto a 10% precast polyacrylamide gel. CFI was detected with OX21 (Table 1) and anti-mouse IgG HRP conjugated antibody (Table 1) diluted in blocking buffer. For nuclei staining, cells were washed 2x with PBS (Gibco) and fixed for 15 minutes with 4% Paraformaldedehyde (Sigma) in PBS and permeabilised for 45 minutes with 1% BSA (Thermo Fisher Scientific)-0.1% Triton X-100 (Sigma) in PBS. Hoechst (Thermo Fisher Scientific) was incubated at 1:5000 in permeabilisation buffer for 15 minutes and nuclei staining was assessed.

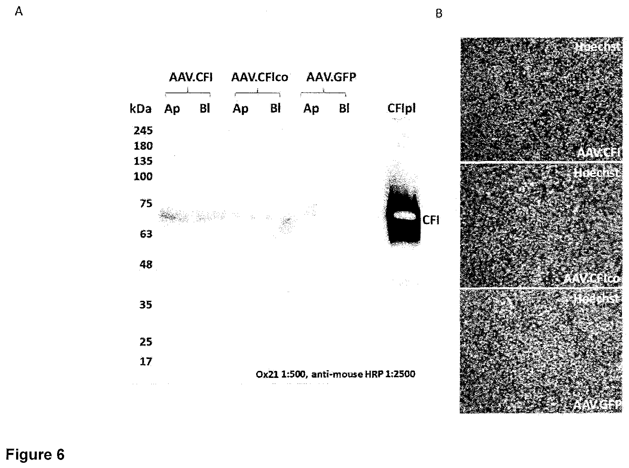

Figure 6 shows immunoblotting of CFI secreted from transwell cultured ARPE-19 cells. Cells were not differentiated to hexagonal cells however they were cultured as a confluent monolayer of cells and cell division was reduced to a minimum by addition of 1% serum medium. 6A: Supernatant from both compartments was loaded under non reducing conditions and western blot analysis was performed using a mouse monoclonal to CFI (Ap=apical compartment and Bl=basolateral compartment). It is shown that CFI is being expressed from a confluent monolayer of cells and that secreted protein is detected in both compartments. 6B: Hoechst staining of nuclei was performed to confirm presence of a monolayer of cells (Staining was performed after harvesting the supernatant).

**In vivo expression analysis of AAV.CFI and AAV.CFlco, subretinal injection of C57/Black 6 mice, analysis of CFI expression by immunoblotting, qPCR and immunohistochemistry.**

### Subretinal injections

Female 8-10 week old C57BL/6J mice (Charles River Laboratories) were used for all experiments. All animals used in this study were treated humanely in accordance with the UK Home Office Regulations under project license 30/3363. Mice were maintained on a 12:12-h light/dark cycle.

Mice were anaesthetised with a mixture of xylazine (10mg/kg)/ketamine(80mg/kg) in sterile saline; pupils were dilated with phenylephrine hydrochloride (2.5 %) and tropicamide (1 %). Proxymetacaine hydrochloride (0.5 %) eye drops were used for additional local anaesthesia. An anterior chamber tap was performed prior to the subretinal injection using sterile, 33G needles (TSK Laboratory) and carbomer gel (Viscotears, Novartis Pharmaceuticals Ltd) and a small circular glass coverslip was used to achieve good visualisation of the fundus. The injection was performed through posterior retina using 10µl NanoFil syringe and 35G bevelled NanoFil needle (World Precision Instruments Ltd). Anaesthesia was reversed with atipamezole (2mg/kg) in sterile saline.

Mice were injected with two different doses (10⁷ genome copies [gc]/eye and 10⁸gc/eye) and two AAV constructs (AAV.CFI and AAV.CFlco). Three mice were injected with a third dose, 10⁹gc/eye of AAV.CFlco; these mice were used for calibration of immunoblots. Virus was diluted in 0.001% PF68 (Gibco) in PBS (Gibco) and sham injections were performed using the same diluent. 12 eyes per condition were subretinally injected.

### Preparation of tissue samples for immunoblotting of CFI

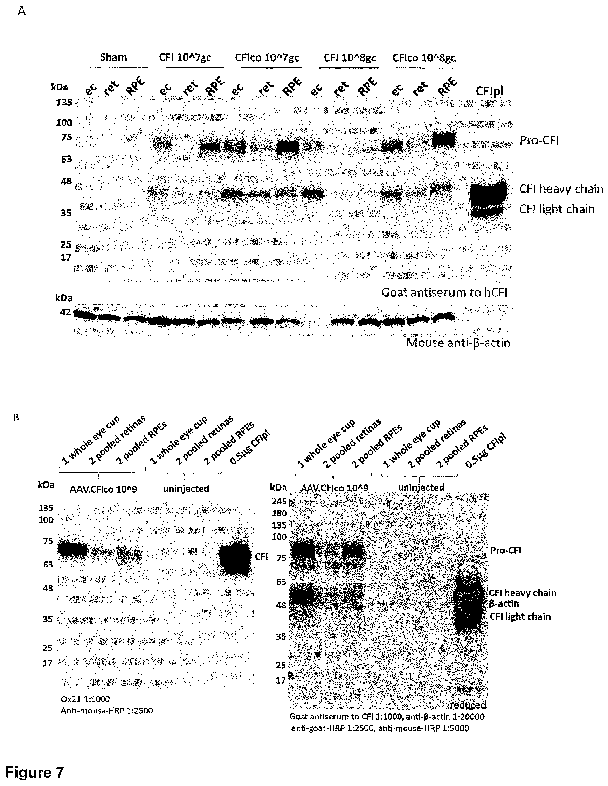

Mice were killed by cervical dislocation 4 weeks post injection. Eyes were removed and prepared as follows. Using a dissecting microscope an incision was made into the cornea. The eye is split into cornea/iris (anterior segment), lens, and posterior eye cup. For a whole eye cup sample, the posterior eye cup is placed in a sterile tube. For some eyes, retina and RPE/choroid/sclera were separated by gently peeling the retina off the RPE/choroid/sclera complex. Samples of three mice per condition were pooled (Figure 7A), except in eyes injected with 10⁹gc/eye of AAV.CFlco (Figure 7B) where samples of two mice were pooled and compared with the non-injected contralateral eye. All samples were immediately put on dry ice to prevent protein degradation and kept at -80°C. For immunoblotting, samples were homogenised in 1xRIPA lysis buffer (Merck Millipore) supplemented with cOmplete^{™} EDTA-free Protease Inhibitor Cocktail (Roche) and cells were disrupted mechanically by mortar and pestle and by sonication. Insoluble protein was centrifuged down and the supernatant aliquoted and stored at -80 °C. Protein concentration was determined using Pierce BCA Protein Assay Kit (Thermo Fisher Scientific) and 40µg protein lysate were loaded in 4x Laemmli buffer (250mM Tris-HCl (pH 6.8), 8% SDS, 40% glycerol, and 0.02% bromophenol blue). Proteins were separated on a 10% precast polyacrylamide gel (Bio-Rad). Proteins were blotted to a PVDF membrane (Bio-Rad) by semi-dry transfer after which the membrane was blocked in blocking buffer (1xTBS pH 8 [Sigma], 0.05% Tween-20 and 5% dried skimmed milk powder [Marvel]). CFI was detected with a) reduced: antiserum to human CFI (Table 1) and anti-goat IgG -HRP conjugated(Table 1) or b) non-reduced: OX21 (Table 1) and donkey anti-mouse IgG HRP conjugated antibody (Abcam) diluted in blocking buffer. For β-actin detection, the membrane was stripped using Restore Western Blot Stripping Buffer (Thermo Fisher Scientific) and reprobed using anti-β-actin (Table 1) and anti-mouse IgG HRP conjugated antibody (Table 1) diluted in blocking buffer. Alternatively, mouse anti-β-actin antibody was used in combination with goat antiserum to CFI.

Figure 7 shows CFI protein expression of pooled samples analysed by immunoblotting. CFI is expressed at detectable levels at all doses and from both AAV.CFI and AAV.CFlco. β-actin was loaded as a loading control. 7A: 40µg protein lysate were loaded under reducing conditions and CFI was detected with a polyclonal goat antiserum to human CFI. CFI is detected as 80kDa (pro-enzyme), 50kDa (processed; heavy chain) and 35kDa (processed; light chain). These bands correspond to the expected size of CFI and confirm processing, i.e. presence of heavy and light chain. 7B: The same amount of protein lysate was also loaded for lysate samples of eyes injected with 10⁹gc/eye of AAV.CFlco or uninjected eyes. CFI was detected with a mouse monoclonal to CFI (left, non-reducing gel) and goat antiserum to CFI (right, reducing gel). The non reducing gel (left) detects CFI as a band at 75kDa in injected animals and no band is detected in the uninjected eye. In the reducing gel (right) CFI appears as 80kDa (pro-enzyme), 50kDa (processed; heavy chain) and 35kDa (processed; light chain).

### Preparation of tissue samples for gene expression analysis

Mice were killed as described above and posterior eye cups were left intact or retina and RPE were separated as described above. Tissue samples were immediately put into sterile tubes containing RNALater (Thermo Fisher Scientific). Samples were kept at 4°C until further processing. RNA was isolated using RNeasy Mini kit (Qiagen) and tissue was homogenised using mortar and pestle in buffer RLT (provided with kit). A complementary treatment with RNase-free DNase (Qiagen) was added to ensure the absence of genomic DNA. 100ng RNA were reverse transcribed to cDNA using Superscript III First Strand Synthesis kit (Thermo Fisher Scientific). After reverse transcription, the reaction was cleaned up using QIAquick PCR Purification Kit (Qiagen). qPCR was conducted on cDNA of one eye per condition (sham, CFI 10⁷gc/eye, CFI 10⁸gc/eye, CFlco 10⁷gc/eye and CFlco 10⁸gc/eye) using a CFX Connect^{™} Real-Time PCR Detection System (Bio Rad). 1.25 ng of cDNA was used per well as template for qPCR reactions with a SYBR green master mix (iTaq Universal SYBR Green Supermix, Bio Rad). Each condition was performed in triplicate; Ct values were obtained using the provided software. Comparative ΔΔCt analysis with mouse beta-actin as a housekeeping gene was used to determine the relative expression of CFI to sham controls.

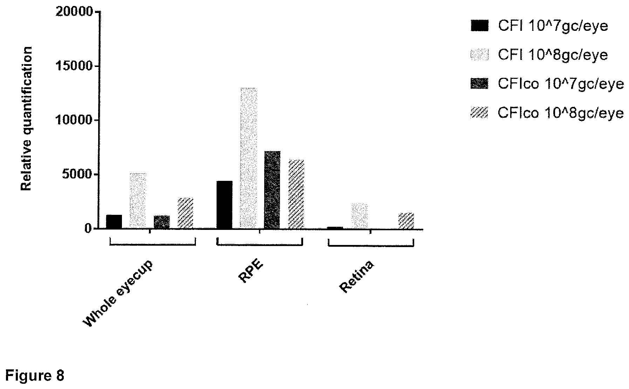

Figure 8 shows gene expression analysis by qPCR. As expected there was no human CFI or CFlco expression in sham injected mice. Since there was only one eye per condition analysed, no statistical analyses could be performed. CFI is expressed from both AAV constructs and expression apparently occurs predominately in the RPE, however injection of 10⁸gc/eye leads to increased CFI mRNA expression in retinal tissue. In the eyecup sample the amount of CFI mRNA to total eyecup mRNA is expected to be smaller than in RPE only, because many areas of the eye will not have expression of CFI; this is reflected by the results.

### Preparation of retinal sections for immunohistochemistry

Mice were killed by cervical dislocation (4 weeks post injection). Eyes were removed and prepared as follows. A hole was made just behind the ora serrata and the eyeball was placed in 4% (w/v) paraformaldehyde in 0.12 M phosphate buffer, pH 7.2 for 1 hour at room temperature. We then removed the anterior segment and the lens and prepared the eyes for sections or whole mount. Eyecups used for sections were cryopreserved with increasing concentrations of sucrose in 0.12 M phosphate buffer, pH 7.2 (10%, for 1 hour at room temperature and 30% overnight at +4°C), embedded in OCT (Tissue-tek, Sakura Finetek USA inc, Ca, USA) and frozen in a mold at -80°C. Sections were cut at a thickness of 10 µm on a cryostat and mounted onto glass slides (Super-Frost, Thermo Fisher Scientific, Waltham, MA, USA). The slides were air dried for 2 hours at room temperature and stored at -80°C. For eyecups used for whole mounts, retina and RPE/choroid were separated by gently peeling the retina off the RPE and stored separately in PBS at +4°C.

### Immunostaining

Sections were blocked and permeabilized by incubation at room temperature for 60 min in PBSGT solution (0.2% (w/v) gelatin, 0.25% (v/v) Triton X-100 in PBS). Whole mounts were blocked and permeabilized by incubation at room temperature for 2 hours in 0.2% (w/v) gelatin, 1.5% (v/v) Triton X-100 in PBS. Subsequently, sections and whole mounts were incubated with primary antibodies (see Table 1) diluted in PBSGT solution overnight at room temperature. After washing in PBST solution (0.1% (v/v) Triton X-100 in PBS), sections and whole mounts were incubated with secondary antibodies coupled to Alexa Fluor594 (Life Technologies, Thermo Fisher Scientific, Waltham, Massachusetts, USA) at a dilution of 1:5000 and stained with DAPI in PBSGT solution for 1.5 h at room temperature. The slides were washed with PBST solution and subsequently cover-slipped with mounting medium (Mowiol, Merck Millipore).

**Table 2. Primary antibodies used in immunohistochemistry in this study.**

| Antibody | Part number | Company | Dilution |
|---|---|---|---|
| rabbit anti-hCFI | HPA024061 | Sigma-Aldrich | 1/250 |
| Alexa Fluor 488 phalloidin | A12379 | Life Technologies | 1/5000 |

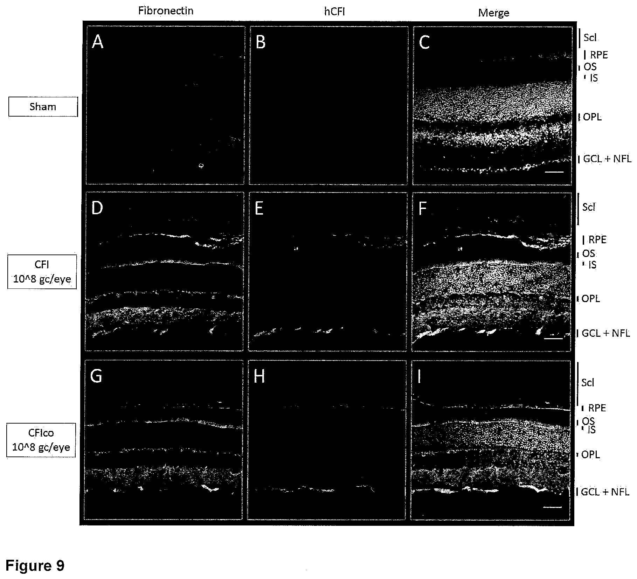

Figure 9 shows fibronectin and hCFI localisation in sham, AAV.CFI and AAV.CFlco injected eyes retinal sections. Fibronectin is used as a co-marker to distinguish the different retinal layers. For both AAV.CFI and AAV.CFlco injected eyes, hCFI is localized in several compartments: slightly in sclera (Scl), strongly in RPE, outer (OS) and inner (IS) segment of photoreceptors, outer plexiform layer (OPL) and presumably in ganglion cell layer (GCL) and nerve fiber layer (NFL). A slight signal in GCL + NFL is visible for sham injected eyes, showing the antibody is not completely specific at these layers.

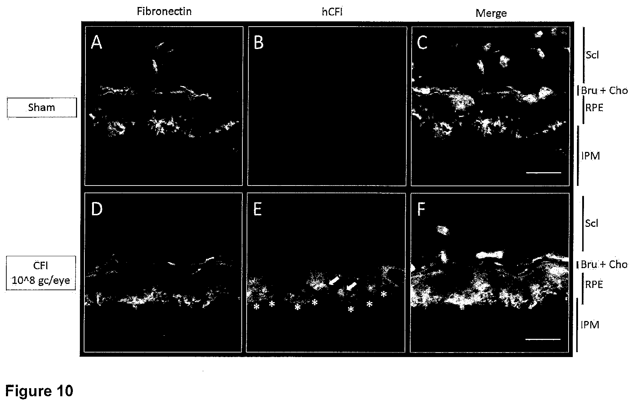

Figures 10 to 13 show higher magnification of the different regions where hCFI is localized for sham and AAV.CFI injected eye retinal sections. The same signal is observed for AAV.CFlco injected eye retinal sections and are not shown.

Figure 10 shows a higher magnification of RPE region for sham and AAV.CFI injected eyes retinal sections. hCFI is localised in the entire RPE layer and in the microvilli of RPE. The staining is vesicular, suggesting a secretory pathway localisation of hCFI.

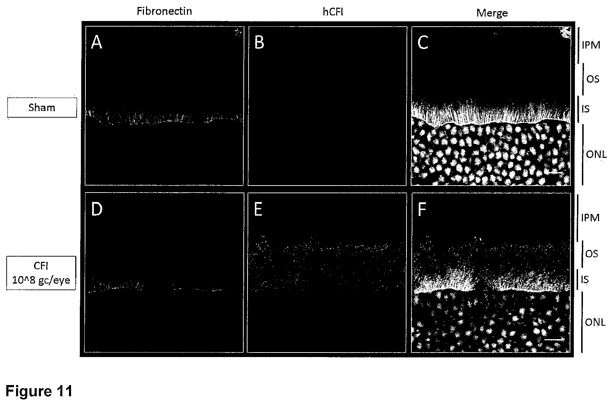

Figure 11 shows a higher magnification of photoreceptor region for sham and AAV.CFI injected eyes retinal sections. hCFI is localised in the inner and outer segments of photoreceptors but was not present in the nuclear/endoplasmic reticulum region of the photoreceptors..

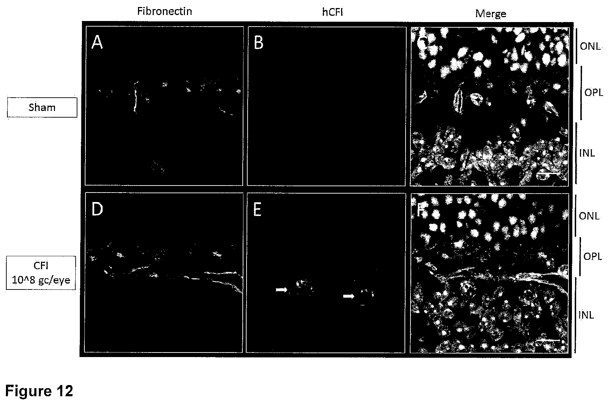

Figure 12 shows a higher magnification of the outer plexiform layer for sham and AAV.CFI injected eyes retinal sections. hCFI is localised in cell bodies suggesting horizontal cell staining. In the absence of a horizontal cell marker, the following references are indicative of how expression in horizontal cells appears in immunohistochemistry: Poche et al (2009), Development, 136:2141-51; Cuenca et al (2010), Exp Eye Res., 91:273-85; Ho et al (2012), PLoS One, 7:e29892.

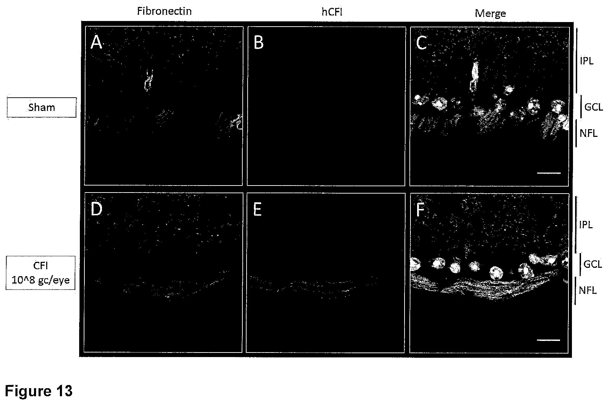

Figure 13 shows a higher magnification of ganglion cell layer region for sham and AAV.CFI injected eyes retinal sections. Although the intensity in nerve fiber layer for hCFI labelling in AAV.CFI injected eyes retinal sections seems to be higher to the intensity for sham injected eyes retinal sections, it is difficult to determine if hCFI is localized in nerve fiber layer.

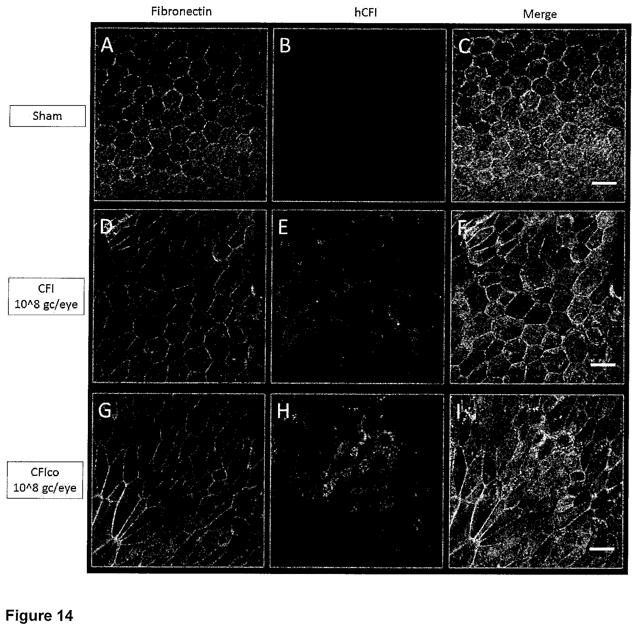

Figure 14 shows fibronectin and hCFI localisation in sham, AAV.CFI and AAV.CFlco injected eyes whole mount RPE. For both AAV.CFI and AAV.CFlco injected eyes, hCFI is localized in RPE cells. The staining is punctuated and seems to be localized in vesicles of the secretory pathway where hCFI is processed.

### Example 2

### In vivo functional assay of ability of AAV.CFI or AAV.CFlco to mitigate light-induced retinal damage

Albino BALB/c mice are housed in the Animal Care Facility under a 12-hour light/12-hour dark cycle with access to food and water ad libitum. The ambient light intensity at the eye level of the animals is 85 ± 18 lux. All experiments are conducted in accordance with the ARVO Statement for the Use of Animals in Ophthalmic and Vision Research and are approved by the Home office.

For the model of geographic atrophy (Bärbel Rohrer; Yao Guo; Kannan Kunchithapautham; Gary S. Gilkeson, Investigative Ophthalmology & Visual Science November 2007, Vol.48, 5282-5289) adult mice (approximately 1 year of age) are moved into a light-treatment room and dark adapted overnight. Animals are housed two animals per cage in clear acrylic glass (Plexiglas; Rohm and Haas, Philadelphia, PA) cages with free access to food and water. Light damage is induced by exposing the animals to 1000 lux of white light provided by two 30-W fluorescent bulbs (T30T12-CW-RS; General Electric, Piscataway, NJ) suspended approximately 40 cm above the cages. Light intensity is measured using a light meter (Extech Instruments, Waltham, MA) to ensure that equal luminance is provided to all animals. This amount of light reduces the numbers of rods to one row within 2 to 3 weeks in albino mice.

To probe the role of Complement Factor I (CFI) in the light-induced loss of outer retinal neurons, mice are exposed to CFI-AAV.CFI (or AAV.CFlco), control GFP-AAV.GFP or sham injection via the subretinal route. Cohorts of 6-8 mice are typically used. Following 3-4 weeks of AAV exposure, light-induced damage is initiated as detailed above. After 2-3 weeks, electroretinography (ERG) is performed. Animals are anesthetized using xylazine and ketamine (20 and 80 mg/kg, respectively), and pupils dilated with 1 drop of phenylephrine HCl (2.5%) and atropine sulfate (1%) and placed on a heated block held at 37°C within a light-tight Faraday cage. Light stimulation is provided using the ERG setup provided by the Micron IV (Phoenix Labs). The optical signal is controlled with mechanical shutters, manually operated neutral density, and a 500-nm bandpass filter. Light intensity per 10-ms flash provided in the stimulus path can be varied in steps of 0.3 log units from 3.0 × 10⁵ to 3.0 × 10¹¹ photons/mm². Scotopic electroretinograms are recorded in response to single-flash stimulation of increasing light intensities, averaging three to five responses. Peak α-wave amplitude is measured from baseline to the initial negative-going voltage, whereas peak b-wave amplitude is measured from the trough of the α-wave to the peak of the positive b-wave. Following ERG, mouse eyes are obtained and processed for histology using hematoxylin and eosin staining and the thickness of outer retinal nuclear layers are quantified.

The expectation is that reducing the activity of the alternative complement pathway using CFI-AAV.CFI/AAV.CFlco exposure will preserve ERG function and reduce outer retinal neuron loss.

In summary, adeno-associated virus is an effective vehicle for enabling sustained expression of Complement Factor I, a regulator of the alternative complement pathway. CFI delivered via AAV has surprisingly been shown to be expressed, correctly processed and secreted in a functionally active form in both active and confluent human RPE cells. These data show that human RPE contain the pro-protein convertases required for secreting CFI in a functional form. In animal translational experiments, sub-retinal delivery of CFI.AAV led to readily detectable expression of a functionally processed form of hCFI produced by mouse RPE cells. Immunostaining also suggests that despite the lack of mouse photoreceptor intracellular expression of hCFI, protein was present in the inner and outer segment region of the photoreceptors, which suggests that RPE secreted hCFI diffuses and could potentially play its critical regulatory role of the complement pathway in a broad region of the chorio-retina. Given the data linking complement dysregulation to age-related macular degeneration, this new therapeutic approach could play a pivotal role in the sustained treatment of this blinding condition.

Although the present invention has been described in connection with specific preferred embodiments, it should be understood that the invention as claimed should not be unduly limited to such specific embodiments.

## Claims

1. An adeno-associated viral (AAV) vector comprising a nucleotide sequence encoding Factor I or a fragment thereof capable of cleaving C3b into iC3b, wherein the nucleotide sequence encoding Factor I or the fragment thereof is operably linked to a polyadenylation site.

2. The AAV vector of claim 1, wherein the nucleotide sequence encoding Factor I or the fragment thereof comprises a sequence selected from the group consisting of:
(a) a nucleotide sequence encoding an amino acid sequence that has at least 70% identity to SEQ ID NO: 1;
(b) a nucleotide sequence that has at least 70% identity to SEQ ID NO: 2; and
(c) the nucleotide sequence of SEQ ID NO: 2.

3. The AAV vector of claim 1 or 2, wherein the viral vector is in the form of a viral particle.

4. The AAV vector of claim 3, wherein the AAV viral particle comprises an AAV2 genome and AAV2 capsid proteins, or an AAV2 genome and AAV5 capsid proteins, or an AAV2 genome and AAV8 capsid proteins.

5. The AAV vector of any preceding claim, wherein the nucleotide sequence encoding Factor I or the fragment thereof is operably linked to a promoter, preferably a retinal-cell specific promoter; preferably wherein the retinal-cell specific promoter is selected from a rhodopsin kinase promoter, a PR2.1 promoter, an RPE65 promoter, and a VMD2 promoter.

6. The AAV vector any preceding claim, wherein the nucleotide sequence encoding Factor I or the fragment thereof is operably linked to a chicken beta-actin (CBA) promoter.

7. The AAV vector of any preceding claim, wherein the nucleotide sequence encoding Factor I or the fragment thereof is operably linked to a CAG promoter, preferably a promoter with the nucleotide sequence of SEQ ID NO: 5.

8. The AAV vector of any preceding claim, wherein the nucleotide sequence encoding Factor I or the fragment thereof is operably linked to a Bovine Growth Hormone poly-A signal, preferably a Bovine Growth Hormone poly-A signal having the nucleotide sequence of SEQ ID NO: 6.

9. An isolated cell transfected with and comprising the AAV vector of any one of claims 1-8.

10. A pharmaceutical composition comprising the AAV vector of any one of claims 1-8 or the isolated cell of claim 9 in combination with a pharmaceutically acceptable carrier, diluent or excipient.

11. The AAV vector, isolated cell or pharmaceutical composition of any one of claims 1-10 for use in treating or preventing an eye disease.

12. The AAV vector, isolated cell or pharmaceutical composition of any one of claims 1-10 for use in treating or preventing a complement-mediated disorder of the eye, wherein the disorder is age-related macular degeneration (AMD) or diabetic retinopathy, preferably AMD; preferably wherein the AMD is dry AMD.

13. The AAV vector, isolated cell or pharmaceutical composition for use according to claim 12, wherein the progression of geographic atrophy is slowed, wherein there is at least a 10% reduction in the increase in geographic atrophy area over the 12 months following administration to a treated eye of a subject, relative to an untreated eye over the same period.

14. The AAV vector, isolated cell or pharmaceutical composition for use according to claim 12 or 13, wherein administration of the AAV vector, isolated cell or pharmaceutical composition increases the level of C3b-inactivating and iC3b-degradation activity in an eye, such as in the retinal pigment epithelium (RPE), of a subject, optionally to a level that exceeds a normal level in an eye or RPE of a subject.

15. The AAV vector, isolated cell or pharmaceutical composition for use according to any one of claims 12-14, wherein the AAV vector, isolated cell or pharmaceutical composition is administered intraocularly.

16. The AAV vector, isolated cell or pharmaceutical composition for use according to any one of claims 12-15, wherein the AAV vector, isolated cell or pharmaceutical composition is administered to the eye of a subject by subretinal, direct retinal, suprachoroidal or intravitreal injection.

17. The AAV vector, isolated cell or pharmaceutical composition for use according to any one of claims 12-16, wherein the AAV vector, isolated cell or pharmaceutical composition is administered to the eye of a subject by subretinal injection.

## Patentansprüche

1. Adeno-assoziiertes-Virus(AAV)-Vektor, umfassend eine Nukleotidsequenz, die Faktor I oder ein Fragment davon mit der Fähigkeit, C3b zu iC3b zu spalten, codiert, wobei die Nukleotidsequenz, die Faktor I oder das Fragment davon codiert, in operativer Verknüpfung mit einer Polyadenylierungsstelle steht.

2. AAV-Vektor nach Anspruch 1, wobei die Nukleotidsequenz, die Faktor I oder das Fragment davon codiert, eine Sequenz umfasst, die ausgewählt ist aus der Gruppe bestehend aus:
(a) einer Nukleotidsequenz, die eine Aminosäuresequenz codiert, die eine Identität von mindestens 70 % mit SEQ ID NO: 1 aufweist,
(b) einer Nukleotidsequenz, die eine Identität von mindestens 70 % mit SEQ ID NO: 2 aufweist, und
(c) der Nukleotidsequenz unter SEQ ID NO: 2.

3. AAV-Vektor nach Anspruch 1 oder 2, wobei der Virusvektor in Form eines Viruspartikels vorliegt.

4. AAV-Vektor nach Anspruch 3, wobei das AAV-Viruspartikel ein AAV2-Genom und AAV2-Kapsidproteine oder ein AAV2-Genom und AAV5-Kapsidproteine oder ein AAV2-Genom und AAV8-Kapsidproteine umfasst.

5. AAV-Vektor nach einem vorhergehenden Anspruch, wobei die Nukleotidsequenz, die Faktor I oder das Fragment davon codiert, in operativer Verknüpfung mit einem Promotor, bevorzugt einem netzhautzellspezifischen Promotor, steht; vorzugsweise wobei der netzhautzellspezifische Promotor aus einem Rhodopsinkinase-Promotor, einem PR2.1-Promotor, einem RPE65-Promotor und einem VMD2-Promotor ausgewählt ist.

6. AAV-Vektor nach einem vorhergehenden Anspruch, wobei die Nukleotidsequenz, die Faktor I oder das Fragment davon codiert, in operativer Verknüpfung mit einem CBA(Chicken Beta-Actin)-Promotor steht.

7. AAV-Vektor nach einem vorhergehenden Anspruch, wobei die Nukleotidsequenz, die Faktor I oder das Fragment davon codiert, in operativer Verknüpfung mit einem CAG-Promotor, bevorzugt einem Promotor mit der Nukleotidsequenz unter SEQ ID NO: 5, steht.

8. AAV-Vektor nach einem vorhergehenden Anspruch, wobei die Nukleotidsequenz, die Faktor I oder das Fragment davon codiert, in operativer Verknüpfung mit einem Bovine Growth Hormone-Poly-A-Signal des Rinderwachstumshormons, bevorzugt einem Bovine Growth Hormone-Poly-A-Signal mit der Nukleotidsequenz unter SEQ ID NO: 6 steht.

9. Isolierte Zelle, transfiziert mit dem und umfassend den AAV-Vektor nach einem der Ansprüche 1-8.

10. Pharmazeutische Zusammensetzung, umfassend den AAV-Vektor nach einem der Ansprüche 1-8 oder die isolierte Zelle nach Anspruch 9 in Kombination mit einem pharmazeutisch unbedenklichen Träger, Verdünnungsmittel oder Exzipienten.

11. AAV-Vektor, isolierte Zelle oder pharmazeutische Zusammensetzung nach einem der Ansprüche 1-10 zur Verwendung bei der Behandlung oder Vorbeugung einer Augenerkrankung.

12. AAV-Vektor, isolierte Zelle oder pharmazeutische Zusammensetzung nach einem der Ansprüche 1-10 zur Verwendung bei der Behandlung oder Vorbeugung einer komplementvermittelten Störung des Auges, wobei die Störung altersbedingte Makuladegeneration (AMD) oder diabetische Retinopathie, bevorzugt AMD ist; vorzugsweise wobei es sich bei der AMD um trockene AMD handelt.

13. AAV-Vektor, isolierte Zelle oder pharmazeutische Zusammensetzung zur Verwendung gemäß Anspruch 12, wobei das Fortschreiten von geografischer Atrophie verlangsamt wird, wobei die Zunahme der geografischen Atrophiezone über die 12 Monate nach Verabreichung an ein behandeltes Auge eines Individuums im Vergleich zu einem unbehandelten Auge über den gleichen Zeitraum um mindestens 10 % reduziert wird.

14. AAV-Vektor, isolierte Zelle oder pharmazeutische Zusammensetzung zur Verwendung gemäß Anspruch 12 oder 13, wobei durch Verabreichung des AAV-Vektors, der isolierten Zelle oder der pharmazeutischen Zusammensetzung das Niveau an C3b-Inaktivierungs- und iC3b-Abbauaktivität in einem Auge, wie etwa im retinalen Pigmentepithel (RPE), eines Individuums erhöht wird, gegebenenfalls auf ein Niveau, das über einem Normalniveau in einem Auge oder RPE eines Individuums liegt.

15. AAV-Vektor, isolierte Zelle oder pharmazeutische Zusammensetzung zur Verwendung gemäß einem der Ansprüche 12-14, wobei der AAV-Vektor, die isolierte Zelle oder die pharmazeutische Zusammensetzung intraokular verabreicht wird.

16. AAV-Vektor, isolierte Zelle oder pharmazeutische Zusammensetzung zur Verwendung gemäß einem der Ansprüche 12-15, wobei der AAV-Vektor, die isolierte Zelle oder die pharmazeutische Zusammensetzung dem Auge eines Individuums durch subretinale, direkte retinale, suprachoroidale oder intravitreale Injektion verabreicht wird.

17. AAV-Vektor, isolierte Zelle oder pharmazeutische Zusammensetzung zur Verwendung gemäß einem der Ansprüche 12-16, wobei der AAV-Vektor, die isolierte Zelle oder die pharmazeutische Zusammensetzung dem Auge eines Individuums durch subretinale Injektion verabreicht wird.

## Revendications

1. Vecteur viral adéno-associé (AAV) comprenant une séquence nucléotidique codant pour le Facteur I ou un fragment de celui-ci capable de cliver C3b en iC3b, dans lequel la séquence nucléotidique codant pour le Facteur I ou le fragment de celui-ci est liée de manière opérationnelle à un site de polyadénylation.

2. Vecteur AAV selon la revendication 1, dans lequel la séquence nucléotidique codant pour le Facteur I ou le fragment de celui-ci comprend une séquence choisie dans le groupe constitué par :
(a) une séquence nucléotidique codant pour une séquence d'acides aminés qui possède au moins 70 % d'identité avec SEQ ID NO : 1 ;
(b) une séquence nucléotidique qui possède au moins 70 % d'identité avec SEQ ID NO : 2 ; et
(c) la séquence nucléotidique de SEQ ID NO : 2.

3. Vecteur AAV selon la revendication 1 ou 2, dans lequel le vecteur viral est sous la forme d'une particule virale.

4. Vecteur AAV selon la revendication 3, dans lequel la particule virale AAV comprend un génome AAV2 et des protéines de capside AAV2, ou un génome AAV2 et des protéines de capside AAV5, ou un génome AAV2 et des protéines de capside AAV8.

5. Vecteur AAV selon l'une quelconque des revendications précédentes, dans lequel la séquence nucléotidique codant pour le Facteur I ou le fragment de celui-ci est liée de manière opérationnelle à un promoteur, de préférence un promoteur spécifique de cellule rétinienne ; de préférence dans lequel le promoteur spécifique de cellule rétinienne est choisi parmi un promoteur de rhodopsine kinase, un promoteur PR2.1, un promoteur RPE65 et un promoteur VMD2.

6. Vecteur AAV selon l'une quelconque des revendications précédentes, dans lequel la séquence nucléotidique codant pour le Facteur I ou le fragment de celui-ci est liée de manière opérationnelle à un promoteur de bêta-actine de poulet (CBA).

7. Vecteur AAV selon l'une quelconque des revendications précédentes, dans lequel la séquence nucléotidique codant pour le Facteur I ou le fragment de celui-ci est liée de manière opérationnelle à un promoteur CAG, de préférence un promoteur ayant la séquence nucléotidique de SEQ ID NO : 5.

8. Vecteur AAV selon l'une quelconque des revendications précédentes, dans lequel la séquence nucléotidique codant pour le Facteur I ou le fragment de celui-ci est liée de manière opérationnelle à un signal poly-A d'Hormone de croissance bovine, de préférence un signal poly-A d'Hormone de croissance bovine ayant la séquence nucléotidique de SEQ ID NO : 6.

9. Cellule isolée transfectée avec et comprenant le vecteur AAV selon l'une quelconque des revendications 1 à 8.

10. Composition pharmaceutique comprenant le vecteur AAV selon l'une quelconque des revendications 1 à 8 ou la cellule isolée selon la revendication 9 en combinaison avec un support, diluant ou excipient pharmaceutiquement acceptable.

11. Vecteur AAV, cellule isolée ou composition pharmaceutique selon l'une quelconque des revendications 1 à 10 pour utilisation dans le traitement ou la prévention d'une maladie oculaire.

12. Vecteur AAV, cellule isolée ou composition pharmaceutique selon l'une quelconque des revendications 1 à 10 pour utilisation dans le traitement ou la prévention d'un trouble de l'œil médié par le complément, dans lequel ou laquelle le trouble est la dégénérescence maculaire liée à l'âge (DMLA) ou la rétinopathie diabétique, de préférence DMLA ; de préférence dans lequel ou laquelle la DMLA est une DMLA sèche.

13. Vecteur AAV, cellule isolée ou composition pharmaceutique pour utilisation selon la revendication 12, dans lequel ou laquelle la progression de l'atrophie géographique est ralentie, dans lequel ou laquelle il y a au moins une réduction de 10 % de l'augmentation de la zone d'atrophie géographique sur les 12 mois suivant l'administration à un œil traité d'un sujet, par rapport à un œil non traité sur la même période.

14. Vecteur AAV, cellule isolée ou composition pharmaceutique pour utilisation selon la revendication 12 ou 13, dans lequel ou laquelle l'administration du vecteur AAV, de la cellule isolée ou de la composition pharmaceutique augmente le niveau d'activité d'inactivation de C3b et de dégradation d'iC3b dans un œil, par exemple dans l'épithélium pigmentaire rétinien (RPE), d'un sujet, facultativement jusqu'à un niveau qui dépasse un niveau normal dans un œil ou RPE d'un sujet.

15. Vecteur AAV, cellule isolée ou composition pharmaceutique pour utilisation selon l'une quelconque des revendications 12 à 14, dans lequel ou laquelle le vecteur AAV, la cellule isolée ou la composition pharmaceutique est administré(e) par voie intraoculaire.

16. Vecteur AAV, cellule isolée ou composition pharmaceutique pour utilisation selon l'une quelconque des revendications 12 à 15, dans lequel ou laquelle le vecteur AAV, la cellule isolée ou la composition pharmaceutique est administré(e) à l'œil d'un sujet par injection sous-rétinienne, rétinienne directe, suprachoroïdienne ou intravitréenne.

17. Vecteur AAV, cellule isolée ou composition pharmaceutique pour utilisation selon l'une quelconque des revendications 12 à 16, dans lequel ou laquelle le vecteur AAV, la cellule isolée ou la composition pharmaceutique est administré(e) à l'œil d'un sujet par injection sous-rétinienne.
